(19) Europäisches Patentamt
European Patent Office
Office européen des brevets

(11) **EP 3 733 633 B1**

(12) **EUROPÄISCHE PATENTSCHRIFT**

(45) Veröffentlichungstag und Bekanntmachung des Hinweises auf die Patenterteilung:
**02.08.2023 Patentblatt 2023/31**

(21) Anmeldenummer: **19020320.8**

(22) Anmeldetag: **03.05.2019**

(51) Internationale Patentklassifikation (IPC):
*C07C 1/20* (2006.01) *C07C 11/04* (2006.01)
*C07C 11/06* (2006.01) *B01J 8/02* (2006.01)
*B01J 8/04* (2006.01) *B01J 19/24* (2006.01)

(52) Gemeinsame Patentklassifikation (CPC):
(C-Sets verfügbar)
**B01J 8/0407; B01J 8/025; B01J 19/2445;**
**C07C 1/20;** B01J 8/02; Y02P 20/52 (Forts.)

(54) **MEHRSTRÄNGIGE ANLAGE UND VERFAHREN ZUR HERSTELLUNG VON OLEFINEN AUS OXYGENATEN**

MULTILINE SYSTEM AND METHOD FOR THE PRODUCTION OF OLEFINS FROM OXYGENATES

INSTALLATION À PLUSIEURS LIGNES ET PROCÉDÉ DE PRODUCTION DES OLÉFINES À PARTIR DES PRODUITS OXYGÉNÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**04.11.2020 Patentblatt 2020/45**

(73) Patentinhaber: **L'AIR LIQUIDE, SOCIÉTÉ ANONYME POUR L'ÉTUDE ET L'EXPLOITATION DES PROCÉDÉS GEORGES CLAUDE**
**75007 Paris (FR)**

(72) Erfinder:
- **Castillo-Welter, Frank**
  **61381 Friedrichsdorf (DE)**
- **Haag, Stéphane**
  **60598 Frankfurt am Main (DE)**
- **Williams, Bryce**
  **60437 Frankfurt am Main (DE)**
- **Drosdzol, Christopher**
  **60438 Frankfurt am Main (DE)**

(74) Vertreter: **Dropsch, Holger**
**Air Liquide Forschung und Entwicklung GmbH**
**Gwinnerstraße 27-33**
**60388 Frankfurt am Main (DE)**

(56) Entgegenhaltungen:
**US-A- 4 404 414 US-A1- 2015 299 067**

(52) Gemeinsame Patentklassifikation (CPC): (Forts.)

C-Sets
**C07C 1/20, C07C 11/04;**
**C07C 1/20, C07C 11/06**

**Beschreibung**

**Gebiet der Erfindung**

Die Erfindung betrifft eine mehrsträngige Anlage zur Herstellung von Olefinen aus Oxygenaten, bei der mehrere Reaktorzüge, die jeweils eine oder mehrere, Katalysator enthaltende Oxygenat-zu-Olefin-(OTO)-Reaktionszonen umfassen, parallel angeordnet und parallel betrieben werden. Dabei werden Vorkehrungen dafür getroffen, dass sich mindestens eine der parallelen Reaktionszonen in einem Regenerierungsmodus betreiben lässt, während in den anderen Reaktionszonen parallel dazu die OTO-Synthesereaktion durchgeführt werden kann. Die aus den einzelnen, im Synthesemodus betriebenen Reaktorzügen erhaltenen Teilproduktströme werden über Teilprodukt-Leitungen ausgeleitet, mittels einer Verbindungsvorrichtung zu einer Gesamtproduktleitung zusammengeführt, mittels eines Verdichters verdichtet und mittels einer mehrstufigen Aufarbeitungsvorrichtung in mehrere olefinhaltige Kohlenwasserstofffraktionen aufgetrennt.

Die Erfindung betrifft ferner ein Verfahren zur Herstellung von Olefinen aus Oxygenaten unter Verwendung einer entsprechenden Anlage.

**Stand der Technik**

Kurzkettige Olefine, insbesondere Propylen (Propen) und Ethylen (Ethen), gehören zu den wichtigsten Grundstoffen der chemischen Industrie. Dies liegt darin begründet, das ausgehend von diesen ungesättigten Verbindungen mit einer kurzen Kettenlänge Moleküle mit langkettigem Kohlenstoffgerüst und zusätzlichen Funktionalisierungen aufgebaut werden können.

Als Quelle für kurzkettige Olefine diente in der Vergangenheit vor allem das Steam-Cracking, d. h. die thermische Spaltung von Kohlenwasserstofffraktionen mit im wesentlichen gesättigten Kohlenwasserstoffen bei der Erdölverarbeitung. In den vergangenen Jahren wurden jedoch weitere Prozesse zur Herstellung von kurzkettigen Olefinen entwickelt. Dies ist zum einen durch steigende Nachfrage bedingt, die durch die vorhandenen Quellen nicht mehr gedeckt werden kann; zum anderen fordert die zunehmende Verknappung von fossilen Rohstoffen die Verwendung anderer Ausgangsstoffe.

Die sog. MTP- (Methanol-to-Propylen) oder auch MTO- (Methanol-to-Olefin) Verfahren zur Herstellung von Propylen und anderen kurzkettigen Olefinen gehen von Methanol als Ausgangsstoff aus. In diesen heterogen katalysierten Verfahren wird aus Methanol zunächst teilweise das Zwischenprodukt Dimethylether (DME) und aus einer Mischung von Methanol und Dimethylether dann nachfolgend im Olefinsynthesereaktor unter Verwendung eines formselektiven, festen Katalysators auf Zeolithbasis bzw. Molekularsiebbasis ein Produktgemisch aus Ethylen, Propylen und den isomeren Butenen als kurzkettige Olefine sowie schwereren Kohlenwasserstoffen mit mehr als vier C-Atomen gebildet. Zudem findet sich im Produktstrom Wasser, welches einerseits aus dem Prozessdampf stammt, der optional dem MTO-Reaktor als Verdünnungsmedium zugeführt wird, sowie aus dem im Synthesereaktor erzeugten Reaktionswasser.

Da außer Methanol und DME gegebenenfalls auch andere kurzkettige, Sauerstoff enthaltenden organischen Moleküle, beispielsweise andere Alkohole als Methanol, als Einsatzstoffe dienen können, wird auch verallgemeinernd von Oxygenat-zu-Olefin-Reaktionen (OTO-Reaktionen) bzw. Oxygenat-zu-Olefin-Synthesen (OTO-Synthesen) gesprochen.

Aufgrund der hohen Exothermie der bei der OTO-Synthese ablaufenden Reaktionen, die zur Bildung unerwünschter Nebenprodukte und zu vorzeitiger Katalysatoralterung bzw. -desaktivierung führen kann, wird es bei der Verwendung von Festbettreaktoren bevorzugt, den festen, körnigen oder stückigen OTO-Synthesekatalysator in einzelnen Katalysatorzonen, Katalysatorschichten oder Katalysatorhorden anzuordnen, zwischen denen eine Zwischenkühlung, beispielsweise durch Einspeisung kalten Einsatzgases bzw. Eduktgases, erfolgen kann. Eine mögliche Ausgestaltung eines solchen Reaktors zeigt die internationale Patentanmeldung WO 2007/140844 A1.

Die sich anschließende Aufreinigung des Produktstroms des Synthesereaktors soll zum einen unerwünschte Nebenprodukte und nicht umgesetzte Edukte abtrennen und die einzelnen Kohlenwasserstofffraktionen möglichst rein herstellen. Üblicherweise wird hierzu im ersten Schritt ein Quenchsystem zur Anwendung gebracht, um durch intensives und rasches Inkontaktbringen eine schlagartige Abkühlung des Produktstroms des Synthesereaktors durch direkten Wärmetausch mit einem fluiden, zumeist flüssigen Quenchmedium, beispielsweise Wasser, zu bewirken. Ein erwünschter Nebeneffekt ist dabei eine gewisse Gaswaschwirkung des typischerweise zumindest teilweise als Gas oder Dampf vorliegenden Produktstroms.

Die offengelegte US-Patentanmeldung US 2015/0299067 A1 beschreibt ein Verfahren zur Herstellung eines olefinischen Produkts bereit, umfassend die Schritte:

(a) Umsetzen eines Oxygenat-Einsatzmaterials in einer Reaktionszone in Gegenwart eines Molekularsieb-Katalysators bei einer Temperatur von 350 bis 1000° C, um einen Reaktionsabflußstrom zu erzeugen, der mindestens Oxygenat, Olefin, Wasser und saure Nebenprodukte umfaßt;

(b) Kühlen des Reaktionsabflußstroms mittels eines indirekten Wärmeaustauschs auf eine Temperatur, die größer ist als die Taupunkttemperatur des Reaktionsabflußstroms;

(c) weiteres schnelles Abkühlen des Reaktionsabflußstroms auf eine Temperatur, die niedriger ist als die Taupunkttemperatur des Reaktionsabflußstroms, durch direkte Injektion einer wäßrigen Flüssigkeit in den Reaktionsabflußstrom, um einen ersten Quenchabflußstrom zu bilden; und

(d) Trennen des ersten Quench-Abflussstroms in einen ersten flüssigen Quench-Abflussstrom und einen ersten gasförmigen Quench-Abflussstrom, der das olefinische Produkt umfasst.

Das US-Patent US 4 404 414 A beschreibt ein Methanol-to Gasoline (MTG)-Umwandlungsverfahren, bei dem die Umwandlung in einer Anzahl von Reaktoren mit Katalysatorbetten durchgeführt wird, die direkt mit dem Einsatzstrom beaufschlagt werden, vorzugsweise in gleichen Anteilen. Ein Verdünnungsgas, vorzugsweise rezyklierte, vom Produkt abgetrennte leichte Kohlenwasserstoffe, wird nacheinander durch jedes der Betten geleitet, um die Reaktionswärme abzuführen. Auf diese Weise wird in jedem Bett ein hohes effektives Rückführungsverhältnis aufrechterhalten, obwohl das tatsächliche Rückführungsverhältnis für das gesamte System niedrig ist.

Ein Beispiel für die einer OTO-Reaktion nachfolgende Aufarbeitung des Synthesereaktorproduktstroms findet sich in der DE 10 2014 112 792 A1, in der beschrieben wird, wie in einem ersten Schritt eine heterogen-katalysierte Umsetzung von wenigstens einem Oxygenat zu einem C2 Olefine, C3 Olefine, C4 Olefine, C5/6 Kohlenwasserstoffverbindungen und C7+ Kohlenwasserstoffverbindungen enthaltenden Produktstrom und in einem zweiten Schritt eine Abtrennung eines zu wenigstens 95 Gew.-% aus C3 Olefinen bestehenden Propylenstroms erzeugt wird.

Die weiteren Aufarbeitungseinheiten, die in der DE 10 2014 112 792 A1 beschrieben werden, entsprechen dem im Stand der Technik üblichen Konzept. Durch das Quenchen kann bereits eine grobe Trennung der Fraktionen in Abhängigkeit von ihrer Kettenlänge der entstehenden Olefine aufgrund des teilweisen Auskondensierens erfolgen, so dass eine flüssige C4+ Fraktion aus dem Quench abgeführt werden kann. Die gasförmig abgetrennte C4- Fraktion wird anschließend in eine Kompressionsstufe eingespeist. Die aus der Kompression stammende C4- Fraktion wird dann einer Trennvorrichtung zugeführt, in der C3- Kohlenwasserstoffe von den C4+ Kohlenwasserstoffen separiert werden. In anschließenden Reinigungsschritten wird die C3 Fraktion in einer weiteren Trenneinrichtung von der C2- Fraktion getrennt, was aufgrund der geringen Siedepunkte der beiden Fraktionen unter Druck erfolgen muss.

Bei der Durchführung der OTO-Synthesereaktion an festen, zeolithbasierten Katalysatoren ist zu beachten, dass - ähnlich wie bei anderen Kohlenwasserstoffumwandlungsreaktionen unter Verwendung solcher Reaktionen, beispielsweise der katalytischen Olefinspaltung - eine kontinuierliche und vergleichsweise rasche Desaktivierung des Katalysators auftritt, die im Zusammenhang mit der Blockierung und/oder Belegung aktiver katalytischer Zentren durch kohlenstoffhaltige, feste Ablagerungen im Zusammenhang steht. Diese daher auch als Verkokung bezeichnete Desaktivierungserscheinung kann großteils durch oxidative Entfernung der Ablagerungen, also durch gezieltes Abbrennen, rückgängig gemacht werden. Die hierzu erforderlichen Reaktionsbedingungen werden beispielsweise für den Fall eines während der katalytischen Olefinspaltung desaktivierten Zeolithkatalysators in der internationalen Patentanmeldung WO 2003/051510 A2 gelehrt. Als Regenerierungsmittel wird dabei oft ein sauerstoffhaltiger Gasstrom, beispielsweise ein mit Stickstoff und/oder Wasserdampf verdünnter Luftstrom verwendet, wobei der Sauerstoffgehalt des Regenerierungsmittels, die Behandlungstemperatur oder beide Parameter schrittweise erhöht werden, um eine möglichst vollständige Entfernung der kohlenstoffhaltigen Ablagerungen unter möglichst vollständigen Bedingungen zu erzielen.

Die Reaktivierung eines bei einer OTO-Synthese desaktivierten Katalysators wird in der Patentveröffentlichung US 2011/0021857 A1 beschrieben. Da es sich hierbei um ein Verfahren mit bewegtem Katalysator handelt, besteht die Möglichkeit, den desaktivierten Katalysator aus einer Reaktions- bzw. Synthesestufe in eine Regenerierungsstufe zu überführen, dort die Regenerierung vorzunehmen und den regenerierten Katalysator anschließend in die Synthesestufe zurückzuführen. Um eine solche Reaktivierung bei Synthesereaktoren vornehmen zu können, die einen festen Katalysator, beispielsweise als Festbett-Schüttung enthalten, ist es dagegen erforderlich, den entsprechenden Reaktor bzw. Reaktorzug von dem Oxygenate enthaltenden Eduktstrom zu trennen, um die oxidative Entkokung durchführen zu können. Um nicht die gesamte Olefin-Produktionsanlage stillsetzen zu müssen, bietet sich daher eine mehrsträngige Ausgestaltung derselben an, bei der bei der mehrere Reaktorzüge, die jeweils eine oder mehrere, Katalysator enthaltende Oxygenat-zu-Olefin-(OTO)-Reaktionszonen umfassen, parallel angeordnet und parallel betrieben werden und bei der sich mindestens eine der parallelen Reaktionszonen in einem Regenerierungsmodus befindet, während in den anderen Reaktionszonen parallel dazu die OTO-Synthesereaktion weiterbetrieben wird. Hierdurch wird eine kontinuierliche Olefinproduktion sichergestellt und die den Reaktorzügen nachgeschalteten Anlagen und Verfahrensstufen können ebenfalls kontinuierlich weiterbetrieben werden. Wichtig ist es dabei, die im Regenerierungsmodus befindlichen Reaktorzüge sicher von den im Synthesemodus befindlichen Reaktorzügen abzutrennen, damit es nicht durch unbeabsichtigte Vermischung des zumeist Sauerstoff enthaltenden Regenerierungsmediums mit kohlenwasserstoffhaltigen Einsatz- oder Produktströmen zur Bildung brennbarer oder sogar explosiver Gasgemische kommt.

EP 3 733 633 B1

Einen wichtigen Aspekt bei der OTO-Synthese bildet auch der Reaktionsdruck. Bei der OTO-Synthese werden in der Regel Kohlenwasserstoffe aufgebaut, die zumeist mehr Kohlenstoffatome enthalten als die als Edukt dienenden Oxygenate wie Methanol oder Dimethylether (DME). Durch die Freisetzung von Wasserdampf als Nebenprodukt verlaufen diese Reaktionen unter Volumenzunahme. Dies wird nachstehend beispielhaft durch die Bruttoreaktionsgleichungen zur Bildung von Ethylen aus Methanol bzw. DME verdeutlicht:

$$2\ CH_3OH = C_2H_4 + 2\ H_2O$$

$$CH_3(O)CH_3 = C_2H_4 + H_2O$$

Zur Erzielung hoher Olefinausbeuten wird es daher bevorzugt, die OTO-Synthese bei möglichst geringem Reaktionsdruck durchzuführen.

Zusammenfassend lässt sich daher schlussfolgern, dass weiterhin Bedarf an verbesserten Olefinsyntheseverfahren besteht, die es erlauben, eine kontinuierliche Olefinproduktion mit hoher Olefinausbeute zu realisieren.

## Beschreibung der Erfindung

Die Aufgabe der Erfindung ist es daher, eine entsprechend verbesserte Anlage zum kontinuierlichen Herstellen von Olefinen aus einem Oxygenate enthaltenden, fluiden Einsatzgemisch und ein korrespondierendes Verfahren bereitzustellen.

Diese Aufgabe wird im Wesentlichen durch eine Anlage mit den Merkmalen des Anspruchs 1 und ein Verfahren mit den Merkmalen des Anspruchs 13 gelöst. Weitere, insbesondere bevorzugte Ausgestaltungen des erfindungsgemäßen Verfahrens und der erfindungsgemäßen Anlage finden sich in den Unteransprüchen der jeweiligen Kategorie.

Als Aufarbeitungsschritte, Aufreinigungsschritte oder Trennschritte sind im Zusammenhang mit der vorliegenden Erfindung grundsätzlich alle Verfahrensschritte zu betrachten, die sich eines thermischen Trennverfahrens bedienen; bevorzugt wird hierbei die Destillation oder Rektifikation verwendet. Dasselbe gilt für die im Zusammenhang mit der Durchführung dieser Schritte stehenden Vorrichtungen und Anlagen.

Unter Fluidverbindung zwischen zwei Bereichen oder Anlagenteilen wird dabei jegliche Art von Verbindung verstanden, die es ermöglicht, dass ein Fluid, beispielsweise ein Reaktionsprodukt oder eine Kohlenwasserstofffraktion, von dem einen zu dem anderen der beiden Bereiche strömen kann, unbeachtlich etwaiger zwischengeschalteter Bereiche, Bauteile oder benötigter Fördermittel. Unter einem Fluid oder fluiden Medium werden dabei Substanzen verstanden, die sich unter dem Einfluss von Scherkräften kontinuierlich verformen, also fließen. Insbesondere sind dies Gase und Flüssigkeiten, aber auch mehrphasige Flüssig-Flüssig-Gemische und Gas-Flüssig-Gemische, wie beispielsweise Gasströmungen mit mitbewegtem Kondensatanteil oder Aerosole.

Unter einem Mittel wird eine Sache verstanden, die die Erreichung eines Zieles ermöglicht oder dabei behilflich ist. Insbesondere werden unter Mitteln zum Durchführen eines bestimmten Verfahrensschrittes alle diejenigen physischen Gegenstände verstanden, die der Fachmann in Betracht ziehen würde, um diesen Verfahrensschritt durchführen zu können. Beispielsweise wird der Fachmann als Mittel zum Einleiten oder Ausleiten eines Stoffstroms alle Transport- und Fördervorrichtungen, also z. B. Rohrleitungen, Pumpen, Verdichter, Ventile, in Betracht ziehen, die ihm aufgrund seines Fachwissens zur Durchführung dieses Verfahrensschrittes notwendig oder sinnvoll erscheinen.

Unter Oxygenaten werden grundsätzlich alle sauerstoffhaltigen Kohlenwasserstoffverbindungen verstanden, die sich unter Oxygenatumwandlungsbedingungen zu Olefinen, insbesondere zu kurzkettigen Olefinen wie Propylen, und weiteren Kohlenwasserstoffprodukten umsetzen lassen. Beispiele für geeignete Oxygenatumwandlungsbedingungen sind dem Fachmann bekannt oder können dem einschlägigen Schrifttum, beispielsweise den eingangs erörterten Patentver-öffentlichungen, entnommen werden.

Als kurzkettige Kohlenwasserstoffe werden im Rahmen der vorliegenden Erfindung insbesondere die Kohlenwasserstoffe verstanden, die bei Umgebungsbedingungen gasförmig vorliegen, beispielsweise, im Falle der Olefine, Ethylen, Propylen sowie die isomeren Butene 1-Buten, cis-2-Buten, trans-2-Buten, iso-Buten.

Als höhere Kohlenwasserstoffe werden im Rahmen der vorliegenden Erfindung insbesondere diejenigen Kohlenwasserstoffe verstanden, die bei Umgebungsbedingungen flüssig vorliegen.

Die genannten Aggregatzustände fest, flüssig und gasförmig bzw. dampfförmig sind immer in Bezug auf die lokalen physikalischen Bedingungen zu verstehen, die bei dem jeweiligen Verfahrensschritt oder in dem jeweiligen Anlagenteil herrschen, sofern nichts anderes angegeben ist. Im Rahmen der vorliegenden Anmeldung sind die Aggregatzustände gasförmig bzw. dampfförmig als synonym zu betrachten. Die Bezeichnung "dampfförmig" dient lediglich zur Verdeutlichung, dass der betreffende Stoff bei Umgebungsbedingungen normalerweise flüssig vorliegt.

Unter einem Auftrennen eines Stoffstroms ist im Zusammenhang mit der vorliegenden Erfindung eine Aufteilung des Stroms in mindestens zwei Teilströme zu verstehen. Wenn nichts anderes angegeben wird, ist davon auszugehen, dass die stoffliche Zusammensetzung der Teilströme derjenigen des Ausgangsstroms entspricht, außer für die Fälle, bei denen dem Fachmann unmittelbar einsichtig ist, dass eine Änderung der stofflichen Zusammensetzung der Teilströme infolge der Bedingungen der Auftrennung zwangsweise auftreten muss, wie dies beispielsweise bei der Destillation der Fall ist.

Unter einer Wärmetransportbeziehung wird ein Vorliegen eines Wärmeaustauschs zwischen zwei Bereichen verstanden, wobei alle Mechanismen des direkten oder indirekten Wärmeaustauschs wie Wärmeleitung, Konvektion oder Wärmestrahlung beteiligt sein können und wobei die betrachteten Bereiche nicht direkt benachbart sein müssen, sondern auch durch Wände oder Zwischenbereiche getrennt sein können.

Unter dem überwiegenden Teil einer Fraktion, eines Stoffstroms etc. ist ein Anteil zu verstehen, der mengenmäßig größer ist als alle anderen jeweils für sich betrachteten Anteile. Insbesondere bei binären Gemischen oder bei der Auftrennung einer Fraktion in zwei Teile ist darunter ein Anteil von mehr als 50 Gew.-% zu verstehen, sofern in konkreten Fall nichts anderes angegeben ist.

Unter der Angabe, dass ein Stoffstrom überwiegend aus einer Komponente oder Komponentengruppe besteht, ist zu verstehen, dass der Stoffmengenanteil (Molenbruch) oder Massenanteil (Massenbruch) dieser Komponente oder Komponentengruppe mengenmäßig größer ist als alle anderen jeweils für sich betrachteten Anteile anderer Komponenten oder Komponentengruppen in dem Stoffstrom. Insbesondere bei binären Gemischen ist darunter ein Anteil von mehr als 50 % zu verstehen. Sofern im konkreten Fall nichts anderes angegeben ist, wird hierbei der Massenanteil (Massenbruch) zugrunde gelegt.

Druckangaben verstehen sich in bar, absolut, abgekürzt bar(a), oder Pascal, absolut, abgekürzt Pa(a) sofern im betreffenden Zusammenhang nichts anderes angegeben ist.

Der Erfindung liegt die Erkenntnis zugrunde, dass es zur Erzielung hoher Zielproduktausbeuten für kurzkettige Olefine wie Ethylen oder Propylen von Bedeutung ist, aufgrund der oben erörterten Druckabhängigkeit der Olefinsynthesereaktionen das Druckniveau in den Reaktionszonen so gering wie möglich zu halten. Da eine Reaktionsführung unter vermindertem Druck bzw. Teilvakuum zu aufwendig und somit unwirtschaftlich ist, bietet sich eine Durchführung der Reaktion bei oder nahe bei Atmosphärendruck an. In der Praxis müssen dabei jedoch stets Druckverluste von Leitungswegen und zwischengeschalteten Bauteilen berücksichtigt werden, so dass zur Erzielung eines ausreichenden Reaktordurchsatzes ein gewisser Überdruck in den Reaktionszonen unvermeidlich ist. Um diesen zu minimieren, ist es insbesondere wichtig, die Druckverluste in den den Reaktionszonen nachgeschalteten Leitungswegen bis hin zum Verdichter zu verringern, da die Summe dieser Druckverluste das minimale Druckniveau des Reaktors und der enthaltenen Reaktionszonen festlegt. Je früher nach Verlassen der Reaktionszonen eine Druckverminderung herbeigeführt werden kann, desto positiver wirkt sich diese daher auf das Druckniveau in den Reaktionszonen aus. Daher ist es von Vorteil, in jedem der einzelnen parallelen Reaktorzüge unmittelbar den Reaktionszonen nachgeordnet eine Wärmerückgewinnungsvorrichtung vorzusehen, auch wenn dies die Investitionskosten gegenüber einer Anlage mit gemeinsamer Wärmerückgewinnungsvorrichtung für alle Reaktorzüge deutlich erhöht. Durch die durch Temperaturerniedrigung bewirkte Volumenverminderung wird der Druck bereits unmittelbar stromabwärts der Reaktionszonen abgesenkt. Dies wird noch durch die Anordnung einer ersten Quenchzone stromabwärts der Wärmerückgewinnungsvorrichtung und stromaufwärts des Verdichters verstärkt, da in dieser eine deutliche Abkühlung bewirkt wird, in deren Folge der Wasseranteil des bzw. der Produktströme weitgehend oder sogar insgesamt kondensiert. Wie eingangs erörtert wurde, findet sich im Produktstrom Wasser, welches einerseits aus dem Prozessdampf stammt, der optional dem OTO-Reaktor als Verdünnungsmedium zugeführt wird, sowie aus dem im Synthesereaktor erzeugten Reaktionswasser. Eine Kondensation dieses Wassergehalts führt daher zu einer deutlichen Volumen- und somit Druckverminderung.

Wie oben ausgeführt, ist es beim Betrieb mehrsträngiger Kohlenwasserstoffsyntheseanlagen, bei denen sich ein oder mehrere Reaktorzüge im Regenerierungsmodus befinden, während in anderen Reaktorzügen parallel dazu die Kohlenwasserstoffsynthese betrieben wird, von besonderer Wichtigkeit, eine sichere Abtrennung oder Absperrung der in den verschiedenen Betriebsarten betriebenen Reaktorzüge vorzunehmen, damit es nicht durch unbeabsichtigte Vermischung des zumeist Sauerstoff enthaltenden Regenerierungsmediums mit kohlenwasserstoffhaltigen Einsatz- oder Produktströmen zur Bildung brennbarer oder sogar explosiver Gasgemische kommt. Dies wird erfindungsgemäß durch das Vorsehen von Absperrvorrichtungen im Leitungsweg der Teilprodukt-Leitungen zum Abtrennen eines Reaktorzugs von nachgeschalteten Anlagenteilen und parallel angeordneten Reaktorzügen erreicht. Als Absperrvorrichtungen können die dem Fachmann bekannten Vorrichtungen verwendet werden, also beispielsweise Ventile, Schieber, Drosselklappen oder Steckscheiben. Das Einbringen einer Absperrvorrichtung in den Leitungsweg einer Teilprodukt-Leitung führt in der Regel zu einem weiteren Anstieg des Druckverlusts, der je nach Art der Absperrvorrichtung unterschiedlich groß sein kann. Deshalb ist es umso wichtiger, die Druckverluste in den den Reaktionszonen nachgeschalteten Leitungswegen bis hin zum Verdichter möglichst weitgehend zu verringern.

**Bevorzugte Ausgestaltungen der Erfindung**

Eine bevorzugte Ausgestaltung der erfindungsgemäßen Anlage ist dadurch gekennzeichnet, dass in jedem Reaktorzug stromabwärts der Wärmerückgewinnungsvorrichtung mindestens zwei parallel angeordnete und parallel betreibbare Teilprodukt-Leitungen zum Ausleiten des Teilproduktstroms aus dem Reaktorzug angeordnet werden, wobei im Leitungsweg jeder der parallelen Teilprodukt-Leitungen mindestens eine Absperrvorrichtung vorhanden ist. Auf diese Weise kann der Druckverlust in diesem Leitungsabschnitt wirkungsvoll verringert werden. Der Vorteil der Verwendung mindestens zweier, bevorzugt zweier parallel angeordneter und parallel betreibbarer Teilprodukt-Leitungen mit jeweils mindestens einer, bevorzugt einer Absperrvorrichtung besteht darin, dass als Absperrvorrichtungen beispielsweise Ventile mit kleinerem Innendurchmesser verwendet werden können, die eine geringere Leckrate aufweisen als Ventile mit größerem Innendurchmesser.

Normalerweise stellen Ventile mit ca. 60 Zoll Innendurchmesser die größten im Handel erhältlichen Ventile in Standardgröße dar. Die Ventilauswahl stellt einen Kompromiss zwischen Größe, Kosten und der Fähigkeit des Ventils dar, Leckagen bei wiederholten Schaltzyklen zu verhindern, wenn es sich in der geschlossenen Position befindet. Im Allgemeinen haben größere Ventile eine höhere Leckrate und sind teurer. Ein vernünftiger Kompromiss zwischen Kosten und Leistung liegt im Bereich von ca. 40 Zoll Ventildurchmesser.

Ventile werden typischerweise basierend auf dem gewünschten Druckabfall spezifiziert. In der vorliegenden Situation ist es wünschenswert, in der offenen Position einen möglichst geringen Druckabfall zu haben. Während viele Ventiltypen verwendet werden können, führt die Anforderung an einen möglichst niedrigen Druckverlust naturgemäß zu einer bevorzugten Auswahl beispielsweise von Ventilen nach Art einer Drossel- oder Absperrklappe. Selbst dann hängt der tatsächliche Druckabfall von der Fluidgeschwindigkeit ab, die durch das Ventil und die angeschlossenen Rohrleitungen fließt.

Um eine besonders sichere Isolierung zwischen dem sauerstoffhaltigen Regenerierungsmittel und den kohlenwasserstoffhaltigen Produktströmen zu erreichen, ist es vorteilhaft, zwei nacheinander angeordnete Ventile zu verwenden. Die Leckrate von zwei Ventilen in Reihe ist im Vergleich zu einem einzigen Ventil erheblich nochmals reduziert und es ist außerdem möglich, den Raum zwischen den beiden Ventilen zu entspannen und an einem sicheren Ort zu entlüften oder mit Inertgas wie Stickstoff zu füllen, wenn der entsprechende Reaktorzug im Regenerierungsmodus betrieben werden soll. Auf diese Weise kann eine besonders sichere Abtrennung der Reaktorzüge erreicht werden, die sich im Synthesemodus bzw. im Regenerierungsmodus befinden. Umso wichtiger ist es dabei aber, dass jedes der in Reihe geschalteten Ventile einen möglichst kleinen Druckverlust aufweist.

Besonders bevorzugt ist es im Hinblick auf die erfindungsgemäße Anlage, dass eine erste Quenchzone zum Durchführen eines direkten Wärmetauschs zwischen einem Teilproduktstrom und einem ersten Quenchmedium umfasst wird, die stromabwärts der Wärmerückgewinnungsvorrichtung und stromaufwärts der Verbindungsvorrichtung, also noch in den Teilprodukt-Leitungen angeordnet ist und dass ferner eine zweite Quenchzone zum Durchführen eines direkten Wärmetauschs zwischen dem Gesamtproduktstrom und einem zweiten Quenchmedium umfasst wird, die stromabwärts der Verbindungsvorrichtung und stromaufwärts des Verdichters, also in der Gesamtproduktleitung angeordnet ist und die mit allen Teilprodukt-Leitungen der einzelnen Reaktorzüge in Fluidverbindung steht. Hierdurch wird eine weitere Druckverminderung noch in den Teilprodukt-Leitungen herbeigeführt, die zu der durch die Wärmerückgewinnungsvorrichtungen bewirkten Druckverminderung hinzutritt. Die durch die erste Quenchzone bewirkte Druckverminderung ist besonders groß, wenn sie so betrieben wird, dass der in den Teilproduktströmen enthaltene Wasserdampf größtenteils, bevorzugt fast vollständig kondensiert. Da - je nach Verwendung von Wasserdampf als Moderator bei der OTO-Synthese - die Teilproduktströme auf Stoffmengenanteile bezogen zum überwiegenden Teil aus Wasserdampf bestehen können, und zusätzlich das bei den Synthesereaktionen als Nebenprodukt gebildete Wasser hinzutritt, wird durch die weitgehende Auskondensation des Wassergehalts der Teilproduktströme eine besonders starke Volumenreduktion und somit eine besonders ausgeprägte Druckverminderung erzielt. Vorteilhaft ist es ferner, dass dann die zweite, in der Gesamtproduktleitung angeordnete Quenchzone so ausgestaltet und betrieben werden kann, dass dort gezielt kondensierbare bzw. absorbierbare Kohlenwasserstoffe abgetrennt werden können. Hierdurch wird nochmals eine Druckverminderung erreicht und die abgetrennten Kohlenwasserstoffe können gezielt der Aufarbeitungsvorrichtung zugeführt werden, wobei sich aufgrund der Vorabtrennung des Wassers in der ersten Quenchzone der Mengenstrom der nunmehr zu behandelnden Flüssigstroms, beispielsweise in einer Flüssig-Flüssig-Phasentrennvorrichtung, deutlich reduziert. Die entsprechenden Vorrichtungen können daher kleiner dimensioniert werden, was zu einer Investitionskostenersparnis führt.

In Bezug auf die letztgenannte Ausgestaltung ist es bevorzugt, dass die erste Quenchzone stromabwärts der Wärmerückgewinnungsvorrichtung und stromaufwärts der Absperrvorrichtung angeordnet ist. Wie zuvor ausgeführt, wird eine besonders deutliche Druckverminderung erzielt, wenn die der Wasserabscheidung dienende erste Quenchzone noch in den Teilprodukt-Leitungen angeordnet wird.

Eine weitere bevorzugte Ausgestaltung der erfindungsgemäßen Anlage ist dadurch gekennzeichnet, dass die mindestens eine Reaktionszone und die stromabwärts der Reaktionszone angeordnete Wärmerückgewinnungsvorrichtung in einem gemeinsamen Behälter angeordnet sind, wobei mittels der stromabwärts der Wärmerückgewinnungsvorrichtung angeordneten Teilprodukt-Leitung der Teilproduktstrom aus dem Behälter ausgeleitet wird. Durch die Anordnung der Reaktionszone und der Wärmerückgewinnungsvorrichtung in einem gemeinsamen Behälter entfällt die Verbindungsleitung zwischen diesen Anlagenteilen und es wird eine weitere Druckverminderung erzielt, da der Leitungsquerschnitt der Verbindungsleitung in der Regel kleiner, und der Druckverlust pro Längeneinheit höher ist als bei dem gemeinsamen Behälter.

In einem weiteren Aspekt der Erfindung ist die Anlage dadurch gekennzeichnet, dass

- die mindestens eine Reaktionszone und die stromabwärts der Reaktionszone angeordnete Wärmerückgewinnungsvorrichtung in einem gemeinsamen Behälter angeordnet sind,
- mittels der stromabwärts der Wärmerückgewinnungsvorrichtung angeordneten Teilprodukt-Leitung der Teilproduktstrom aus dem Behälter ausgeleitet wird,
- alle Teilprodukt-Leitungen der Reaktorzüge mittels der Verbindungsvorrichtung zusammengeführt werden,
- die erste Quenchzone zwischen der Verbindungsvorrichtung und dem Verdichter angeordnet ist und mit allen Teilprodukt-Leitungen der einzelnen Reaktorzüge in Fluidverbindung steht.

Für die Anordnung mehrerer Anlagenteile in einem gemeinsamen Behälter werden die oben erörterten Vorteile erhalten. In Kombination mit den anderen Merkmalen dieser Ausgestaltung wird eine besonders effektive Druckverminderung erzielt.

In einem weiteren Aspekt ist die erfindungsgemäße Anlage dadurch gekennzeichnet, dass

- die mindestens eine Reaktionszone, die stromabwärts der Reaktionszone angeordnete Wärmerückgewinnungsvorrichtung und die stromabwärts der Wärmerückgewinnungsvorrichtung angeordnete erste Quenchzone in einem gemeinsamen Behälter angeordnet sind,
- mittels der stromabwärts der ersten Quenchzone angeordneten Teilprodukt-Leitung der Teilproduktstrom aus dem Behälter ausgeleitet wird,
- alle Teilprodukt-Leitungen der Reaktorzüge mittels der Verbindungsvorrichtung zusammengeführt werden,
- die zweite Quenchzone zwischen der Verbindungsvorrichtung und dem Verdichter angeordnet ist und mit allen Teilprodukt-Leitungen der einzelnen Reaktorzüge in Fluidverbindung steht.

Für die Anordnung mehrerer Anlagenteile in einem gemeinsamen Behälter werden die oben erörterten Vorteile erhalten. In Kombination mit den anderen Merkmalen dieser Ausgestaltung wird eine besonders effektive Druckverminderung erzielt.

In einem weiteren Aspekt ist die erfindungsgemäße Anlage dadurch gekennzeichnet, dass die mindestens eine Reaktionszone, die stromabwärts der Reaktionszone angeordnete Wärmerückgewinnungsvorrichtung, die stromabwärts der Wärmerückgewinnungsvorrichtung angeordnete erste Quenchzone und die stromabwärts der ersten Quenchzone angeordnete zweite Quenchzone in einem gemeinsamen Behälter angeordnet sind,

- mittels der stromabwärts der zweiten Quenchzone angeordneten Teilprodukt-Leitung der Teilproduktstrom aus dem Behälter ausgeleitet wird,
- alle Teilprodukt-Leitungen der Reaktorzüge mittels der Verbindungsvorrichtung zusammengeführt werden.

Für die Anordnung mehrerer Anlagenteile in einem gemeinsamen Behälter werden die oben erörterten Vorteile erhalten. In Kombination mit den anderen Merkmalen dieser Ausgestaltung wird eine besonders effektive Druckverminderung erzielt.

In Bezug auf die Ausgestaltungen der erfindungsgemäßen Anlage mit gemeinsamem Behälter ist es bevorzugt, wenn der gemeinsame Behälter mit einer Leitung zum Ausleiten eines flüssigen Kondensats verbunden ist. Für die Anordnung mehrerer Anlagenteile in einem gemeinsamen Behälter werden die oben erörterten Vorteile erhalten. Falls es bereits in der Wärmerückgewinnungsvorrichtung zur Kondensatabscheidung kommt, ist es günstig, das Kondensat über die Leitung zum Ausleiten desselben abzuführen, da es beim Auftreten einer Zweiphasenströmung gas/flüssig zu Fluktuationen beim Anlagenbetrieb aufgrund von Druckschwankungen, Mitrisseffekten und ähnlichem kommen kann. Dasselbe gilt in noch stärkerem Maße für Ausgestaltungen mit einer oder mehreren Quenchzonen, die ebenfalls in dem gemeinsamen Behälter angeordnet sind, da hierbei zwangsweise eine Flüssigphase anfällt, die aus dem gemeinsamen Behälter ausgeleitet werden muss.

In Bezug auf die Ausgestaltungen der erfindungsgemäßen Anlage mit gemeinsamem Behälter ist es ferner bevorzugt, dass die Wärmerückgewinnungsvorrichtungen als Plattenwärmetauscher ausgestaltet sind. Plattenwärmetauscher sind konstruktiv einfach, unempfindlich gegenüber Verstopfungen, weisen gute Wärmeübertragungseigenschaften auf und bewirken nur einen geringen Druckverlust pro Längeneinheit, weswegen sie vorteilhaft mit den anderen Ausgestaltungsmerkmalen zusammenwirken, die ebenfalls auf die Verringerung des Druckverlusts abzielen.

In einem weiteren Aspekt ist die letztgenannte Ausgestaltung der erfindungsgemäßen Anlage dadurch gekennzeichnet, dass die Wärmerückgewinnungsvorrichtungen mindestens zwei Plattenwärmetauscher umfassen, die mit unterschiedlichen Kühlfluiden betrieben werden. Auf diese Weise kann eine besonders effektive Wärmeabfuhr in Analogie zu einem Gegenstrom-Wärmetauscher erreicht werden. Als Kühlfluide können kalte Prozessmedien und/oder dedizierte Kühlmittel verwendet werden. Wenn im ersten Kühlschritt ein kaltes Prozessmedium, beispielsweise der oxygenathaltige Einsatzstrom, und im zweiten Kühlschritt ein weiteres Kühlmittel verwendet wird, kann die Erwärmung des Prozessmediums bis zu der erwünschten Temperatur für den nachfolgenden Einsatz erfolgen und auf diese begrenzt werden, da die restliche Kühlwirkung im zweiten Kühlschritt erfolgt.

In einem weiteren Aspekt sind die Ausgestaltungen der erfindungsgemäßen Anlage mit gemeinsamem Behälter dadurch gekennzeichnet, dass die Reaktionszonen in einer Wärmetransportbeziehung mit Plattenwärmetauschern stehen, mittels derer die Reaktionszonen durch indirekten Wärmetausch mit einem Kühlfluid gekühlt werden. Die Gesamtwärmetönung der bei der OTO-Synthese ablaufenden Reaktionen ist stark exotherm. Deshalb ist es wichtig, die insbesondere am Katalysator freigesetzte Wärme rasch und nahe am Freisetzungsort abzuführen, damit es nicht zu Schädigungen und vorzeitiger und womöglich irreversibler Desaktivierung des Katalysators kommt. Dies wird durch diese Ausgestaltungen erreicht.

Eine bevorzugte Ausgestaltung des erfindungsgemäßen Verfahrens ist dadurch gekennzeichnet, dass die Anlage drei Reaktorzüge umfasst, von denen zwei mit Oxygenate enthaltendem, fluiden Einsatzgemisch beaufschlagt werden und einer parallel dazu mit einem gasförmigen sauerstoffhaltigen Regenerierungsmittel beaufschlagt wird. Untersuchungen zeigen, dass mit dieser Anordnung, bezogen auf die Anlagengröße und die hiermit verbundenen Investitionskosten, eine wirtschaftlich günstige und kontinuierliche Olefinproduktion sichergestellt wird. Sobald die Regenerierung in einem Reaktorzug abgeschlossen wurde, steht er wieder für die Olefinproduktion zur Verfügung und einer der weiteren Reaktorzüge kann in den Regenerierungsmodus überwechseln, sobald die Aktivität des enthaltenen Katalysators unter ein Mindestniveau fällt. Somit stehen zu jedem Zeitpunkt zwei Reaktorzüge für die Olefinproduktion zur Verfügung und die nachgeschalteten Anlagenteile können kontinuierlich betrieben werden, was insbesondere für die Aufarbeitungsvorrichtung bedeutend ist, da diese aufgrund der Vielzahl der einzelnen Trennoperationen und -vorrichtungen lange Zeit für das Erreichen eines stationären Zustands benötigt und solange noch keine spezifikationsgerechten Produkte hergestellt werden können.

In einem weiteren Aspekt ist das erfindungsgemäße Verfahren ist dadurch gekennzeichnet, dass , dass mindestens 40 %, bevorzugt mindestens 70 % des Produktgases vor der Absperrvorrichtung als Kondensat abgeschieden werden. Untersuchungen haben gezeigt, dass bei einem Abscheidegrad von mindestens 70 % des Produktgases als Kondensat, vor allem als Wasser, eine besonders deutliche Druckverminderung, eine erhöhte Propylenausbeute und eine Volumenentlastung der nachgeschalteten Anlagenteile erhalten wird. Ein Abscheidegrad von mindestens 40 % des Produktgases stellt dagegen einen günstigen Kompromiss zwischen den oben genannten Vorteilen einerseits und dem Abscheideaufwand andererseits dar, der sich in Form von benötigter Wärmetauscherfläche bzw. Kühlmittelbedarf widerspiegelt.

**Ausführungs- und Zahlenbeispiele**

Weitere Merkmale, Vorteile und Anwendungsmöglichkeiten der Erfindung ergeben sich aus der nachfolgenden Beschreibung von Ausführungsbeispielen und der Zeichnungen. Dabei bilden alle beschriebenen und/oder bildlich dargestellten Merkmale für sich oder in beliebiger Kombination den Gegenstand der Erfindung, unabhängig von ihrer Zusammenfassung in den Ansprüchen oder deren Rückbezügen.

Es zeigen:

Fig. 1    eine schematische Darstellung einer ersten Ausgestaltung,
Fig. 2    eine schematische Darstellung einer zweiten Ausgestaltung,
Fig. 3    eine schematische Darstellung einer dritten Ausgestaltung,
Fig. 4    eine schematische Darstellung einer vierten Ausgestaltung,
Fig. 5    eine schematische Darstellung einer fünften Ausgestaltung

jeweils der erfindungsgemäßen Anlage bzw. des erfindungsgemäßen Verfahrens, wobei die bildliche Darstellung auf den Anlagenteil bzw. Verfahrensteil stromabwärts des Verdichters beschränkt ist. Es schließen sich in jeder der gezeigten Ausgestaltungen jeweils der Verdichter und die stromabwärts des Verdichters angeordnete, mehrstufige Aufarbeitungsvorrichtung zum Auftrennen des verdichteten Gesamtprodukts in mehrere olefinhaltige Kohlenwasserstofffraktionen an.

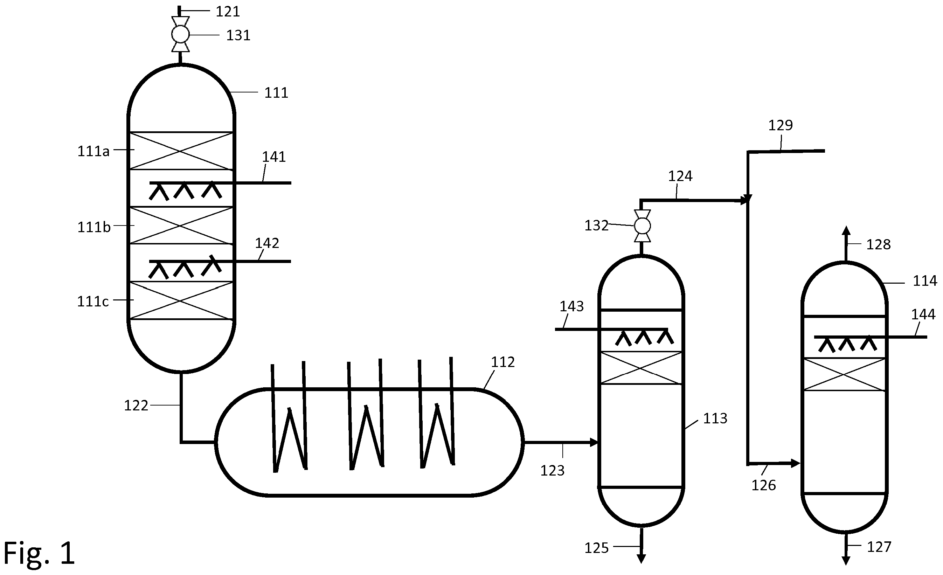

Fig.1 zeigt einen schematischen Aufbau eines Reaktorzuges nach einer ersten Ausgestaltung der Erfindung. Der Reaktorzug beinhaltet die OTO-Reaktionszone 111, eine Wärmerückgewinnungsvorrichtung 112 und eine erste Quenchzone 113. Die Vorrichtungen sind durch die Leitungen 122 und 123 miteinander verbunden. Eine zweite Quenchzone 114 wird von mehreren parallel geschalteten Reaktorzügen gemeinsam genutzt, was durch die Leitung 129 angedeutet ist. In dem vorliegenden Beispiel umfasst die Anlage drei separate Reaktorzüge mit drei den Reaktorzügen zugeordneten ersten Quenchzonen. Absperrvorrichtungen 131 und 132 sind am Eintritt und am Austritt aus dem Reaktorzug in den Leitungen 121 und 124 vorgesehen.

Über Leitung 121 wird durch eine eintrittsseitige Absperrvorrichtung 131 ein Oxygenate enthaltender Eduktstrom in die OTO-Reaktionszone 111 eingebracht. Diese weist im konkreten Fall beispielhaft drei mit festem, körnigem, formselektivem, kommerziell erhältlichem Zeolithkatalysator gefüllte Katalysatorfestbetten 111a, 111b und 111c auf, was jedoch lediglich symbolisch für eine beliebige Anzahl Katalysatorfestbetten zu verstehen ist. Die Einspeisung von zusätzlichem kühlerem Eduktstrom auf jedes nachgeschaltete Festbett ist mittels der Leitungen 141 und 142 angedeutet.

Da der Betrieb des OTO-Reaktors bei möglichst niedrigen Drücken die Propylenausbeute verbessert, ist der Druckabfall der dem Reaktor stromabwärts nachgeschalteten Anlagen, Rohrleitungen und anderer Bauteile wie z. B. Ventile für die Propylenausbeute wichtiger als der Druckabfall entsprechender Bauteile stromaufwärts des OTO-Reaktors. Je deutlicher daher der Druckverlust in dem Anlagenteil zwischen dem Ausgang aus der OTO-Reaktionszone und dem Eingang des Verdichters gesenkt werden kann, umso vorteilhafter wirkt sich dies für die Ausbeute an den Zielprodukten, beispielsweise Propylen, aus.

Bei einer Ausgestaltung einer OTO-Syntheseanlage gemäß Fig. 1 im technischen Maßstab beträgt die Jahreskapazität typischerweise 470 kta Propylen. Dabei beträgt der Volumenstrom des Produktgases am Ausgang der OTO-Reaktionszone 230 kNm$^3$/h bei einer Temperatur von 480 °C und einem Druck von 130 kPa(a). Das Produktgemisch tritt nach Verlassen der Wärmerückgewinnungsvorrichtung in eine erste Quenchzone ein, bei der Prozesswasser das gasförmige Produktgemisch mit einem Ausgangsdruck von 118 kPa(a) auf 55 °C abkühlt, wodurch auch erhebliche Wassermengen kondensieren.

Zusammengenommen senkt die Kombination von niedrigerer Temperatur und Kondensation den tatsächlichen Volumenstrom von 495 km$^3$/h am Ausgang des MTP-Reaktors auf 76 km$^3$/h am Ausgang der ersten Quenchvorrichtung. Nach den üblichen Rohrbemessungsverfahren entsprechen die genannten Durchflüsse Rohr- bzw. Ventildurchmessern von 106 Zoll am Ausgang der OTO-Reaktionszone, 86 Zoll am Ausgang der Wärmerückgewinnungsvorrichtung und 56 Zoll am Ausgang der ersten Quenchzone. Da der Handel Standardgrößen für automatisch betriebene Ventile mit Größen unter 60 Zoll bereithält, kann somit für die austrittsseitige Absperrvorrichtung 132 ein handelsübliches automatisches Ventil verwendet werden. Ein solches automatisches Ventil ist ein betätigtes Ventil, das durch mechanische Mittel oder Instrumentenluft gesteuert wird, wodurch zumindest eine Fernsteuerung von Ventilöffnungs-/Schließvorgängen über ein Kontrollsystem möglich ist. Die Ventilauswahl stellt einen Kompromiss dar zwischen Größe, Kosten und der Fähigkeit des Ventils, bei wiederholten Schaltspielen in der geschlossenen Stellung Leckagen zu vermeiden. Die Ventile werden typischerweise basierend auf dem gewünschten Druckabfall spezifiziert. Zudem sollte der Druckverlust in der voll geöffneten Stellung möglichst gering sein.

Als Referenzwert für die Strömungsgeschwindigkeit des Produktgases kann für eine Ausgestaltung gemäß Fig. 1 eine Gasgeschwindigkeit von 13 m/s in der Absperrvorrichtung 132 zugrunde gelegt werden. Für eine gegebene Ventilgröße kann die Änderung des Druckabfalls aus der Änderung der Geschwindigkeit gemäß

$$(\Delta p_2/\Delta p_1) = (v_2/v1)^2$$

bestimmt werden, wobei
$p_x$ die konkrete Gasgeschwindigkeit beim Durchfluss durch das geöffnete Ventil und
$\Delta p_x$ der einzustellende Druckverlust ist.

Eine Erhöhung von 13 auf 16 m/s würde beispielsweise eine Erhöhung des Druckverlustes von etwa 50 % über das Ventil ergeben, was die kritische Wechselwirkung zwischen der Größe der Absperrvorrichtung und dem Ziel der Minimierung des OTO-Reaktordrucks zur Erzielung hoher Propylenausbeuten veranschaulicht.

Um beim Wechsel der Betriebsarten eines Reaktorzuges vom Synthesebetrieb in den Regenerierungsbetrieb eine zuverlässige und sichere Trennung zwischen sauerstoffhaltigen Strömen und brennbaren, kohlenwasserstoffhaltigen Gasen zu erreichen, können auch zwei Automatikventile als Absperrvorrichtungen hintereinander geschaltet werden. Die Leckrate wird hierdurch im Vergleich zu einem einzelnen Ventil erheblich reduziert. Darüber hinaus ist es mit diesem Konzept möglich, den Raum zwischen den beiden Absperrvorrichtungen drucklos zu machen und/oder mit Inertgas zu belüften, ggf. auch mit Überdruck. Hierdurch wird gewährleistet, dass im Falle von Undichtigkeit lediglich Inertgas in die Anlage eindringt, jedoch kein brennbares Gas unbeabsichtigt in die Umgebung abgegeben wird.

Das in der ersten Quenchzone abgekühlte und teilkondensierte Produktgas wird über Leitung 124 und die im Leitungsweg 124 angeordnete Absperrvorrichtung 132 sowie Gesamtproduktleitung 126 zu der zweiten Quenchzone geleitet. Über den beispielhaft gezeigten Leitungsweg 125 mündet der Teilproduktstrom des parallelen, nicht gezeigten Reaktorzuges ein, der sich ebenfalls im Synthesebetrieb befindet, während sich ein dritter Reaktorzug im parallel im Regenerierungsmodus befindet und über die in diesem Reaktorzug befindliche Absperrvorrichtung von den beiden im Synthesemodus befindlichen Reaktorzügen abgetrennt ist.

In der zweiten Quenchzone erfolgt eine weitere Abkühlung des Gesamtproduktgases mit Wasser als Quenchmedium, wobei nunmehr vor allem schwerere, hochsiedende Kohlenwasserstoffe kondensieren und die leichteren, niedrigsiedenden Kohlenwasserstoffe in der Gasphase verbleiben. Letztere werden über Leitung 128 zu einem Verdichter und nachfolgend zu einer mehrstufigen Aufarbeitungsvorrichtung zum Auftrennen des verdichteten Gesamtprodukts in mehrere olefinhaltige Kohlenwasserstofffraktionen geführt (Verdichter und Aufarbeitungsvorrichtung bildlich nicht dargestellt). Über Leitung 127 wird das wässrige Quenchmedium aus der zweiten Quenchzone ausgeleitet. Die schwereren Kohlenwasserstoffe bilden eine separate, organische Flüssigphase und werden über eine nicht gezeigte Leitung ebenfalls dem Verdichter und der nachgeschalteten Aufarbeitungsvorrichtung zugeführt. Alternativ können die schwereren Kohlenwasserstoffe auch ohne weitere Aufarbeitung in einem Benzinprodukt gesammelt werden.

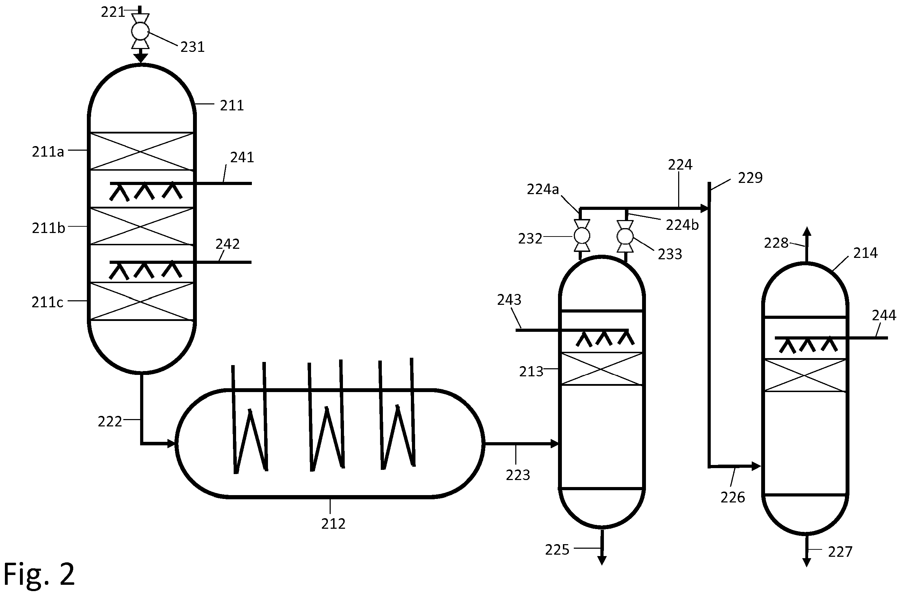

Figur 2 zeigt eine zweite Ausführungsform der vorliegenden Erfindung. Das aus der OTO-Reaktionszone 211 austretende Produktgemisch wird über Leitung 222 in eine Wärmerückgewinnungsvorrichtung 212 mit vorzugsweise mehreren Wärmetauschern geführt. Anschließend leitet Leitung 223 den abgekühlten Teilproduktstrom in eine erste Quenchzone 213, in die über Leitung 243 das Quenchmedium, bevorzugt Wasser, eingebracht wird

Von dieser ersten Quenchvorrichtung 213 wird das gebrauchte Quenchmedium über Leitung 225 ausgeleitet und das abgekühlte Produktgemisch über zwei parallele Leitungen 224a und 224b und den zugehörigen beiden Absperrvorrichtungen 232 und 233, bevorzugt als Ventile ausgestaltet, geführt, bevor es dann über Leitung 224 ausgeleitet und über Leitung 229 mit Strömen aus anderen, parallel angeordneten Reaktorzügen zu einem Gesamtproduktstrom vereinigt und über Leitung 226 in die zweite Quenchzone eingebracht wird. In dieser wird über Leitung 227 eine flüssige Fraktion und über Leitung 228 eine gasförmige Fraktion abgeführt. Das Quenchmedium, bevorzugt Wasser, wird wie angedeutet über Leitung 244 eingebracht. Die weiteren Eigenschaften und die weitere Aufarbeitung der verschiedenen Produktströme der zweiten Quenchzone entsprechen den im Zusammenhang mit Fig. 1 erläuterten.

Die Aufteilung des Austrittsstroms aus der ersten Quenchzone 213 auf zwei parallel geschaltete Absperrvorrichtung führt dazu, dass die Größe dieser beiden Vorrichtungen, bevorzugt die Größe zweier Ventile, deutlich reduziert werden kann. So kann bei der Verwendung von Ventilen deren Größe von ca. 56 Zoll auf 40 Zoll reduziert werden. Die Kosten und die Leckrate von zwei Automatikventilen der Größe 40 Zoll werden im Vergleich zu Ventilen der Größe 56 Zoll erheblich reduziert, wobei der Druckverlust durch die Aufteilung auf zwei Teilströme und die damit verbundene Reduzierung der Strömungsgeschwindigkeit tendenziell abnimmt und im schlechtesten Falle gleichbleibt. Demnach werden mit dieser Ausgestaltung Vorteile hinsichtlich der Anlagensicherheit und der Investitionskosten bei gleicher oder verbesserter Propylenausbeute erhalten.

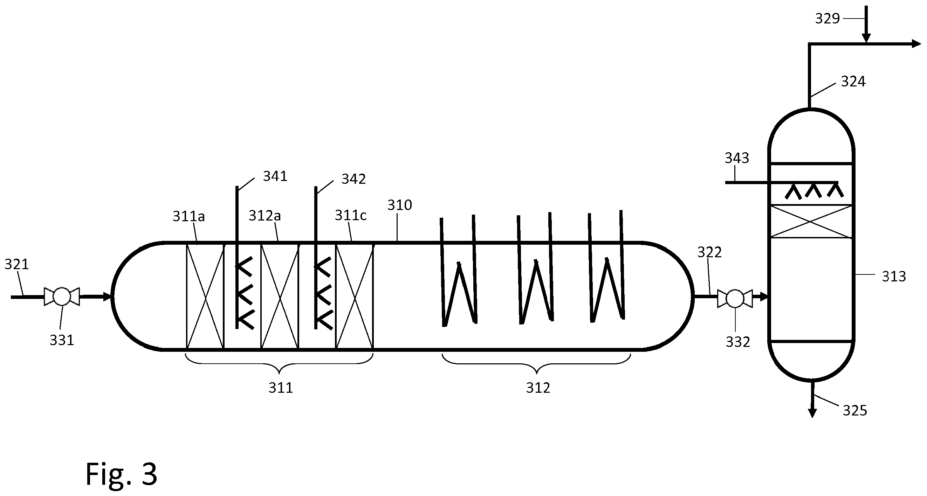

Figur 3 zeigt eine dritte Ausgestaltung der Erfindung. Hier befindet sich innerhalb eines gemeinsamen Behälters 310 eine Reaktionszone 311, der sich in Strömungsrichtung eine Wärmerückgewinnungsvorrichtung 312 anschließt. Über Leitung 321 und die in ihrem Leitungsweg integrierte, eintrittsseitige Absperrvorrichtung 331 wird der Eduktteilstrom in den gemeinsamen Behälter 310 eingebracht und passiert dort zuerst die Reaktionszone 311. Diese umfasst auch hier exemplarisch drei Katalysatorfestbetten 311a, 311b und 311c. Die Einspeisung von zusätzlichem kühlerem Eduktstrom auf jedes nachgeschaltete Katalysatorfestbett ist wiederum mittels der Leitungen 341 und 342 angedeutet.

Der aus der Reaktionszone austretende Teilproduktstrom wird dann innerhalb des gemeinsamen Behälters 310 in eine Wärmerückgewinnungsvorrichtung 312 geführt. Danach verlässt der gekühlte Teilproduktstrom über Leitung 322 den gemeinsamen Behälter 310 und passiert die zweite Absperrvorrichtung 332, bevor er - gemeinsam mit den Teilproduktströmen der parallelen, nicht dargestellten Reaktorzüge - in die erste Quenchzone 313 eingebracht wird, welcher über Leitung 343 das Quenchmedium, bevorzugt Wasser, zugeführt wird. Aus dieser Quenchzone wird über die Leitungen 324 und 325 die gasförmige und die flüssige Fraktion abgezogen. Die weiteren Eigenschaften und die weitere Aufarbeitung der verschiedenen Produktströme der Quenchzone entsprechen den im Zusammenhang mit der zweiten Quenchzone in Fig. 1 erläuterten. Mittels Leitung 329 ist wiederum die Verschaltung mit den weiteren, parallel betriebenen Reaktorzügen angedeutet.

In diesem Beispiel umfasst der Reaktorzug nur die Reaktionszone und die Wärmerückgewinnungsvorrichtung innerhalb des gemeinsamen Behälters. In der Regel erfordert diese Anordnung nur ein gemeinsames Quenchsystem für alle parallelen Reaktorzüge. Eine solche Konfiguration ist beispielsweise in den folgenden Beispielen besonders vorteilhaft:

(a) Der die Reaktionszone verlassende Teilproduktstrom wird mittels der Wärmerückgewinnungsvorrichtung durch Dampferzeugung, Methanolverdampfung, Kohlenwasserstoffrecycling-Verdampfung/Überhitzung und/oder andere Prozess- oder Nutzströme auf eine Temperatur von ca. 70 °C gekühlt. In diesem Fall wird der aus dem gemeinsamen Behälter 310 austretende, gekühlte Teilproduktstrom aufgrund der teilweisen Kondensation von 330 km$^3$/h bei 190 °C vor der Kondensation auf nur 67 km$^3$/h nach der teilweisen Kondensation der enthaltenen Dämpfe aufgrund der Kühlung reduziert, was etwa einem Stoffmengenanteil von 73 mol-% entspricht (Flüssigkeitsauslass aus Behälter 310 nach der Abkühlung nicht gezeigt). Damit lassen sich auch bei großen Anlagengrößen für die Absperrvorrichtung verhältnismäßig übliche Ventilgrößen, beispielsweise 54 Zoll verwenden.

(b) Die Größe der Syntheseanlage wird von 470 kta auf eine Produktionskapazität von 100 kta reduziert. In diesem Fall reduziert sich der aus dem gemeinsamen Behälter 310 austretende Teilproduktstrom von 330 km$^3$/h x 100/470

auf 70 km$^3$/h bei 190 °C. Damit sinkt ebenfalls die Anforderung an die Ventilgröße.

(c) Eine weitere Möglichkeit ist die Reduzierung der Anlagenkapazität von 470 kta auf eine Zwischengröße von 200 kta. In diesem Fall ist die Kühlung des die Reaktionszone verlassenden Teilproduktstroms mittels der Wärmerückgewinnungsvorrichtung durch Dampferzeugung, Methanolverdampfung, Kohlenwasserstoffrecycling-Verdampfung/Überhitzung und/oder andere Prozess- oder Nutzströme auf 90 °C erforderlich, um etwa 39 % des Dampfes (auf molarer Basis) zu kondensieren. Der verbleibende Gasvolumenstrom nach teilweiser Kondensation der enthaltenen Dämpfe von 67 km$^3$/h ermöglicht dann erneut eine Nutzung von handelsüblichen Ventilgrößen, insbesondere 54 Zoll.

In allen Ausgestaltungen der Erfindung mit gemeinsamem Behälter für mehrere Anlagenteile, also den in den Figuren 3, 4, 5 gezeigten Ausgestaltungsbeispielen, ist es möglich, diesen Aspekt mit der Aufteilung des Teilproduktstroms auf zwei parallel angeordnete verschaltete Absperrvorrichtungen zu kombinieren, wie es in Figur 2 gezeigt ist.

Der Hauptvorteil dieser Ausführungsform gemäß Fig. 3 besteht darin, dass der Druckverlust zwischen der Reaktionszone und der Wärmerückgewinnungsvorrichtung im Vergleich zu den in Fig. 1 und 2 gezeigten Ausgestaltungen weiter minimiert ist, da Leitungen zwischen diesen beiden Baugruppen entfallen.

Eine Ausgestaltung der Erfindung gemäß Fig. 3 weist gegenüber der Ausgestaltung gemäß Figur 1 deutliche Druckverlustreduzierungen auf. So beträgt der Druckverlust für die Verbindungsleitung 123 zwischen der Reaktionszone und der Wärmerückgewinnungsvorrichtung 1,2 mbar und der Druckverlust über die Wärmerückgewinnungsvorrichtung beträgt 2,7 mbar, in Summe daher 3,9 mbar. Für dieselbe Anordnung gemäß Figur 3 resultiert ein Druckverlust von 1,4 mbar, die Druckverlustersparnis beträgt somit 2,5 mbar, entsprechend einer Verminderung des Druckverlustes um 63 % gegenüber einer Ausgestaltung gemäß Figur 1. Dies entspricht aufgrund der Verbesserung der Propylenausbeute von 1,2 % auf relativer Basis. Umgerechnet auf eine Anlagenkapazität von 470 kta bedeutet dies, dass bei gleichbleibendem Edukteinsatz die Propylenproduktion um 5,8 kta gesteigert werden kann.

Die in Fig. 3 gezeigte, horizontale, liegende Anordnung des gemeinsamen Behälters 310 ist nur beispielhaft zu verstehen. Je nach Aufstellungsort und verfügbarem Platz wäre auch eine vertikale, stehende Anordnung des gemeinsamen Behälters 310 denkbar und ggf. vorteilhaft.

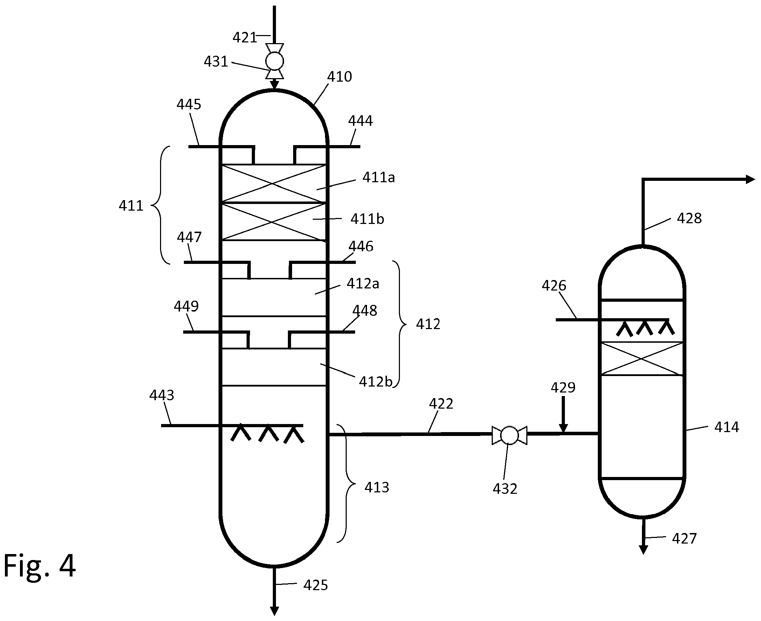

Figur 4 zeigt eine vierte Ausführungsform der vorliegenden Erfindung. Innerhalb des gemeinsamen Behälters 410 befindet sich nun neben der Reaktionszone 411 und der Wärmerückgewinnungsvorrichtung 412 auch eine erste Quenchzone 413.

Die Reaktionszone umfasst hier exemplarisch die beiden Katalysatorfestbetten 411a, 411b, die mittels Plattenwärmetauschern gekühlt werden, in die über Leitungen 444, 445 ein Kühlfluid ein- bzw. ausgeleitet wird. Auf diese Weise kann die freigesetzte Reaktionswärme der exothermen OTO-Synthesereaktionen effizient abgeführt werden. Jede andere Ausgestaltung der Reaktionszone ist möglich.

Der Reaktionszone nachgeschaltet ist die Wärmerückgewinnungsvorrichtung 412 mit beispielsweise zwei Wärmetauschern 412a und 412b, die vorzugsweise ebenfalls als Plattenwärmetauscher ausgestaltet sind, die die Zu- und Ableitungen 446, 447, 448 und 449 für ihr jeweiliges Kühlfluid aufweisen.

Der Wärmerückgewinnungsvorrichtung nachgeschaltet befindet sich auch die erste Quenchzone 413 im gemeinsamen Behälter 410, in die über Leitung 443 das Quenchmedium eingebracht wird. Die erste Quenchzone wird vorzugsweise so betrieben, dass ein Großteil des im Teilproduktstrom enthaltenen Wassers abgeschieden und über Leitung 425 ausgeleitet werden kann. Eine Absperrvorrichtung 432 ist in der Leitung 422 angeordnet, mittels der der abgekühlte und teilkondensierte Produktstrom aus dem Reaktorzug ausgeleitet wird. Über Leitung 422 erfolgt die Einleitung des Teilproduktstroms in eine zweite Quenchzone 414, gemeinsam mit den Teilproduktströmen der parallelen, bildlich nicht dargestellten Reaktorzüge. Aus dieser Quenchzone wird über die Leitungen 428 und 427 die gasförmige und die flüssige Fraktion abgezogen. Die weiteren Eigenschaften und die weitere Aufarbeitung der verschiedenen Produktströme der Quenchzone entsprechen den im Zusammenhang mit der zweiten Quenchzone in Fig. 1 erläuterten.

In dieser Ausgestaltung der Erfindung umfasst der Reaktorzug die Reaktionszone, die Wärmerückgewinnungsvorrichtung und die erste Quenchzone innerhalb des gemeinsamen Behälters. Eine solche Konfiguration ist beispielsweise in den folgenden Beispielen besonders vorteilhaft:

(a) Der die Reaktionszone verlassende Teilproduktstrom wird zunächst mittels der Wärmerückgewinnungsvorrichtung durch indirekten Wärmetausch abgekühlt, wobei die abgeführte Wärmemenge zur Dampferzeugung, Methanolverdampfung, Kohlenwasserstoffrecycling-Verdampfung/Überhitzung und/oder zur Aufheizung andere Prozessoder Nutzströme genutzt wird. Anschließend erfolgt in der ersten Quenchzone eine weitere Abkühlung durch direkten Wärmetausch auf eine Temperatur von ca. 70 °C. In diesem Fall wird der Gasstrom aufgrund der teilweisen Kondensation von 330 km$^3$/h bei 190 °C vor der Kondensation auf nur 67 km$^3$/h nach der teilweisen Kondensation der enthaltenen Dämpfe reduziert. Dies entspricht einer Kondensation des Dampfes von ca. 73 mol-%, wodurch auch für Anlagen mit einer Produktionskapazität 470 kta übliche Ventile, insbesondere in einer Größe von 54 Zoll eingesetzt werden können und sich der durch den Staudruck bedingte Druckverlust durch den wegfallenden Anteil des Volumenstroms entsprechend verringert.

(b) Die Größe der Syntheseanlage wird von 470 kta auf 120 kta reduziert. In diesem Fall beträgt der Strom nach Passieren der Wärmerückgewinnungsvorrichtung proportional gemäß 330 km$^3$/h x 120/470 rund 84 km$^3$/h bei 190 °C. Durch Passieren der ersten Quenchzone sinkt die Gastemperatur weiter von 190 °C auf unter 100 °C, der Gasvolumenstrom beträgt dann 70 km$^3$/h. Damit liegen die für den Fall (a) beschriebenen Vorteile auch hier vor.

Durch die Integration von Reaktionszone, Wärmerückgewinnungsvorrichtung und erster Quenchzone in einen gemeinsamen Behälter wird der Druckverlust gegenüber den zuvor erörterten Beispielen weiter abgesenkt. Insbesondere weist eine Ausgestaltung der Erfindung gemäß Figur 4 gegenüber der Ausgestaltung gemäß Figur 1 weitere deutliche Druckverlustreduzierungen auf. So beträgt der Druckverlust für die Verbindungsleitung 123 zwischen der Reaktionszone und der Wärmerückgewinnungsvorrichtung 1,2 mbar und der Druckverlust über die Wärmerückgewinnungsvorrichtung beträgt 2,7 mbar, in Summe daher 3,9 mbar. Ferner weist die erste Quenchzone in Fig. 1 einen Druckverlust von 11,2 mbar bzw. 8,4 mbar in der Ein- und Ausgangsverrohrung auf. Ausgehend von diesem kumulierten Druckverlust von insgesamt 23,6 mbar erspart die Ausgestaltung gemäß Fig. 4 insgesamt 22,3 mbar, entsprechend einer Reduzierung um 94 %. Eine solche Verringerung Druckverlustes führt bei gleichbleibendem Edukteinsatz zu einer Erhöhung der Propylenausbeute um 3,8 %, was bei einer Anlagenkapazität von 470 kta einer Zusatzausbeute von 17,9 kta Propylen entspricht.

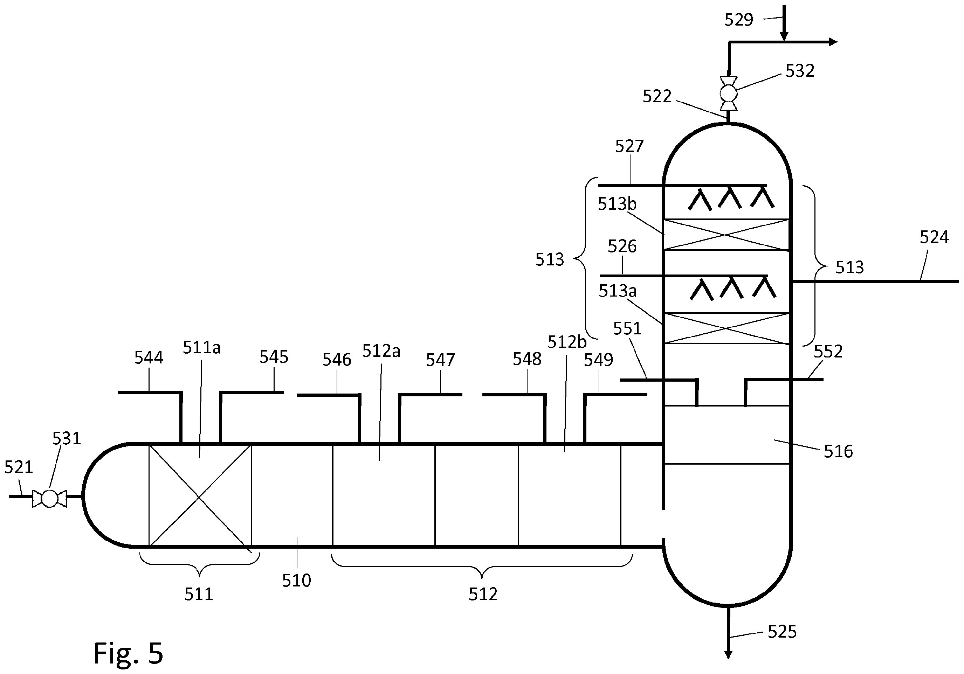

Figur 5 zeigt schließlich eine fünfte Ausführungsform der vorliegenden Erfindung. Sie beinhaltet die Anordnung der Reaktionszone 511, der Wärmerückgewinnungsvorrichtung 512 sowie der als Doppelquench ausgestalteten Quenchzone 513 mit der ersten Quenchzone 513a und der zweiten Quenchzone 513b in einem gemeinsamen Behälter 510.

Über Leitung 521 und die in ihrem Leitungsweg integrierte, eintrittsseitige Absperrvorrichtung 531 wird der Eduktteilstrom in den gemeinsamen Behälter 510 eingebracht und passiert dort zuerst die Reaktionszone 511. Die Reaktionszone umfasst hier exemplarisch das Katalysatorfestbett 511a, das mittels eines Plattenwärmetauschers gekühlt wird, in den über Leitungen 544, 545 ein Kühlfluid ein- bzw. ausgeleitet wird. Auf diese Weise kann die freigesetzte Reaktionswärme der exothermen OTO-Synthesereaktionen effizient abgeführt werden. Jede andere Ausgestaltung der Reaktionszone ist möglich. Bevorzugt umfasst die Reaktionszone mehrere hintereinandergeschaltete Katalysatorfestbetten.

Der Reaktionszone nachgeschaltet ist die Wärmerückgewinnungsvorrichtung 512 mit beispielsweise zwei Wärmetauschern 512a und 512b, die vorzugsweise ebenfalls als Plattenwärmetauscher ausgestaltet sind, die die Zu- und Ableitungen 546, 547, 548 und 549 für ihr jeweiliges Kühlfluid aufweisen. Danach folgt ein Wechsel der Strömungsrichtung um bevorzugt 90°, wobei - je nach den Gegebenheiten am Aufstellungsort - auch eine Ausgestaltung ohne oder mit einem anderen Wechsel der Strömungsrichtung denkbar ist. Allerdings ist die gezeigte, stehende Anordnung des Anlagenteils stromabwärts des Wärmetauschers 512b vorteilhaft, da in diesem Teil verstärkt Kondensate und Quenchmedien anfallen und daher leichter gesammelt und über Leitung 525 ausgeleitet werden können.

Im konkreten Fall wird ein weiterer Wärmetauscher 516 mit zugehörigen Ein- und Auslassleitungen 551 und 552 für das Kühlmedium eingesetzt, welcher auch der Wärmerückgewinnungsvorrichtung 512 zuzuordnen ist. Alternativ wird dieser Wärmetauscher 516 an einer anderen Stelle oder gar nicht vorgesehen.

Im Anschluss passiert das gekühlte Reaktionsgemisch das Quenchsystem 513 mit der ersten Quenchzone 513a und der zweiten Quenchzone 513b, die jeweils über Leitung 526 und 527 mit Quenchmedium versorgt werden. Über Leitung 525 und optional auch Leitung 524 wird eine oder werden mehrere flüssige Phase(n) abgezogen. Die in der Figur gezeigte Leitung 524 ist nur exemplarisch und bezüglich der Höhe ihrer Anordnung variabel zu verstehen. Falls über Leitung 524 eine zweite flüssige Phase abgezogen werden soll, empfiehlt es sich, zwischen der ersten und er zweiten Quenchzone einen bildlich nicht dargestellten Trennboden, beispielsweise einen Kaminboden, vorzusehen, der für die Gasphase durchlässig, aber für die zweite flüssige Phase undurchlässig ist, so dass sich diese auf dem Trennboden sammelt und über Leitung 524 ausgeleitet werden kann.

Über Leitung 522 und die im Leitungsweg 522 angeordnete Absperrvorrichtung 532 wird die gasförmige Fraktion ausgeleitet. Das Einmünden der Teilproduktströme der weiteren, parallel betriebenen Reaktorzüge ist wieder symbolisch über Leitung 529 angedeutet. Die weiteren Eigenschaften und die weitere Aufarbeitung der verschiedenen Produktströme der Quenchzone entsprechen den im Zusammenhang mit der zweiten Quenchzone in Fig. 1 erläuterten.

In dieser Ausgestaltung der Erfindung umfasst der Reaktorzug die Reaktionszone, die Wärmerückgewinnungsvorrichtung und beide Quenchzonen innerhalb des gemeinsamen Behälters. Eine solche Konfiguration ist beispielsweise in den folgenden Beispielen besonders vorteilhaft:

(a) Der die Reaktionszone verlassende Teilproduktstrom wird zunächst mittels der Wärmerückgewinnungsvorrichtung durch indirekten Wärmetausch auf eine Zwischentemperatur von 100 bis 160 °C abgekühlt, bevor die Kondensation der Flüssigkeit stattfindet. Anschließend wird durch zusätzliche Wärmetauscher, beispielsweise den Wärmetauscher 516, die Gastemperatur unter den Kondensationspunkt abgesenkt. Hierbei ist es günstig, dass durch die Anordnung des Wärmetauscher 516 im vertikalen Teil des gemeinsamen Behälters das erzeugte Kondensat nach unten abfließen kann, so dass es gemeinsam mit dem gebrauchten Quenchmedium über Leitung 525 ausgeleitet werden kann. Anschließend erfolgt in der ersten Quenchzone eine weitere Abkühlung durch direkten Wärmetausch auf eine Temperatur von ca. 70 °C.

Anschließend erfolgt eine weitere Temperaturabsenkung auf eine Temperatur von 50 bis 95 °C in der ersten Quenchzone und schließlich auf eine Temperatur von 40 bis 50 °C in der zweiten Quenchzone. In diesem Beispiel wird der Teilproduktgasstrom aufgrund der teilweisen Kondensation von 330 km$^3$/h (basierend auf einer Anlagenkapazität von 470 kta) bei 190 °C vor der Kühlung und Kondensation auf nur 48 km$^3$/h reduziert, was einer Kondensation von 79 mol-% des Dampfes entspricht, wodurch auch für Anlagen mit einer Produktionskapazität 470 kta übliche Ventile, insbesondere in einer Größe von 45 Zoll eingesetzt werden können und sich der durch den Staudruck bedingte Druckverlust durch den wegfallenden Anteil des Volumenstroms entsprechend verringert.

(b) In Fortführung des unter (a) erörterten Beispiels könnte der Teilprodukt-Volumenstrom auf 42 km$^3$/h reduziert werden, wenn eine Austrittstemperatur von 40 °C und somit eine Kondensation von 81 mol-% des Dampfes erzielt werden. Dies würde sogar Ventilgrößen von 42 Zoll ermöglichen. Auch hier ist es möglich, diesen Aspekt mit der Aufteilung des Teilproduktstroms auf zwei parallel angeordnete verschaltete Absperrvorrichtungen zu kombinieren, wie es in Figur 2 gezeigt ist, so dass Ventilgrößen von 30 oder 32 Zoll verwendet werden könnten.

Durch die Integration von Reaktionszone, Wärmerückgewinnungsvorrichtung und beider Quenchzonen in einen gemeinsamen Behälter wird der Druckverlust gegenüber den zuvor erörterten Beispielen nochmals weiter abgesenkt. Insbesondere weist eine Ausgestaltung der Erfindung gemäß Figur 5 gegenüber der Ausgestaltung gemäß Figur 4 eine weitere Druckverlustreduzierung von 9,3 mbar auf, so dass der Gesamtdruckverlust von 32,8 mbar (Reaktionszone, Wärmerückgewinnungsvorrichtung und beide Quenchzonen in jeweils separaten Behältern mit Verbindungsleitungen) um insgesamt 31,5 mbar, also um 96 %, reduziert werden kann. Folglich kommt es zu einer Erhöhung der Propylenausbeute von 4,8 %, was bei einer Anlagenkapazität von 470 kta einer Steigerung von 22,6 kta entspricht.

**Bezugszeichenliste**

| | |
|---|---|
| 111 | Reaktionszone |
| 111a - 111c | Katalysatorfestbett |
| 112 | Wärmerückgewinnungsvorrichtung |
| 113 | erste Quenchzone |
| 114 | zweite Quenchzone |
| 121 - 129 | Leitung |
| 131, 132 | Absperrvorrichtung |
| 141 - 144 | Leitung |
| | |
| 211 | Reaktionszone |
| 211a - 211c | Katalysatorfestbett |
| 212 | Wärmerückgewinnungsvorrichtung |
| 213 | erste Quenchzone |
| 214 | zweite Quenchzone |
| 221 - 229 | Leitung |
| 231 - 233 | Absperrvorrichtung |
| 241 - 244 | Leitung |
| | |
| 310 | gemeinsamer Behälter |
| 311 | Reaktionszone |
| 311a - 311c | Katalysatorfestbett |
| 312 | Wärmerückgewinnungsvorrichtung |
| 313 | erste Quenchzone |
| 321 - 329 | Leitung |
| 331, 332 | Absperrvorrichtung |
| 341 - 343 | Leitung |

| 410 | gemeinsamer Behälter |
| 411 | Reaktionszone |
| 411a - 411b | Katalysatorfestbett mit Wärmetauschern |
| 412 | Wärmerückgewinnungsvorrichtung |
| 412a - 412b | Wärmetauscher |
| 413 | erste Quenchzone |
| 414 | zweite Quenchzone |
| 421 - 429 | Leitung |
| 431, 432 | Absperrvorrichtung |
| 443 - 449 | Leitung |

| 510 | gemeinsamer Behälter |
| 511 | Reaktionszone |
| 511a | Katalysatorfestbett mit Wärmetauscher |
| 512 | Wärmerückgewinnungsvorrichtung |
| 512a, 512b | Wärmetauscher |
| 513 | Quenchsystem |
| 513a | erste Quenchvorrichtung |
| 513b | zweite Quenchvorrichtung |
| 521 - 529 | Leitung |
| 531, 532 | Absperrvorrichtung |
| 544 - 552 | Leitung |

**Patentansprüche**

1. Mehrsträngige Anlage zum Herstellen von Olefinen aus einem Oxygenate enthaltenden, fluiden Einsatzgemisch, umfassend folgende, miteinander in Fluidverbindung stehende Bestandteile und Baugruppen:

    (a) mindestens zwei parallel angeordnete und parallel betreibbare Reaktorzüge, wobei jeder Reaktorzug umfasst:

        (a1) mindestens eine Oxygenat-zu-Olefin-(OTO)-Reaktionszone, enthaltend einen für die Umsetzung der Oxygenate zu Olefinen unter Oxygenatumwandlungsbedingungen aktiven und selektiven Katalysator, Mittel zum Zuführen des Oxygenate enthaltenden Einsatzgemischs zu der Reaktionszone, Mittel zum Ausleiten eines Olefine enthaltenden Teilproduktstroms aus der Reaktionszone, Mittel zum Zuführen eines gasförmigen sauerstoffhaltigen Regenerierungsmittels zu der Reaktionszone, Mittel zum Ausleiten eines Kohlenoxide enthaltenden Regenerierungsabgases aus der Reaktionszone,
        (a2) eine stromabwärts der Reaktionszone angeordnete Wärmerückgewinnungsvorrichtung, umfassend mindestens einen Wärmetauscher zum Durchführen eines indirekten Wärmetauschs zwischen dem aus der Reaktionszone abgeführten Produktgas und einem Kühlfluid,
        (a3) eine stromabwärts der Wärmerückgewinnungsvorrichtung angeordnete Teilprodukt-Leitung zum Ausleiten des Teilproduktstroms aus dem Reaktorzug,
        (a4) eine Absperrvorrichtung im Leitungsweg der Teilprodukt-Leitung zum Abtrennen eines Reaktorzugs von nachgeschalteten Anlagenteilen und parallel angeordneten Reaktorzügen,

    (b) eine stromabwärts der einzelnen Reaktorzüge angeordnete Verbindungsvorrichtung für das Zusammenführen der einzelnen Teilprodukt-Leitungen zu einer Gesamtproduktleitung,
    (c) einen stromabwärts der Verbindungsvorrichtung angeordneten Verdichter zum Verdichten des mittels der Gesamtproduktleitung herangeführten Gesamtprodukts,
    (d) eine stromabwärts des Verdichters angeordnete, mehrstufige Aufarbeitungsvorrichtung zum Auftrennen des verdichteten Gesamtprodukts in mehrere olefinhaltige Kohlenwasserstofffraktionen,
    (e) wobei ferner mindestens eine erste Quenchzone zum Durchführen eines direkten Wärmetauschs zwischen einem oder mehreren Teilproduktströmen oder dem Gesamtproduktstrom und einem ersten Quenchmedium umfasst wird, wobei die erste Quenchzone stromabwärts der Wärmerückgewinnungsvorrichtung und stromaufwärts des Verdichters angeordnet ist.

2. Anlage nach Anspruch 1, **dadurch gekennzeichnet, dass** in jedem Reaktorzug stromabwärts der Wärmerückgewinnungsvorrichtung mindestens zwei parallel angeordnete und parallel betreibbare Teilprodukt-Leitungen zum Ausleiten des Teilproduktstroms aus dem Reaktorzug angeordnet werden, wobei im Leitungsweg jeder der parallelen Teilprodukt-Leitungen mindestens eine Absperrvorrichtung vorhanden ist.

3. Anlage nach Anspruch 1 oder 2, **dadurch gekennzeichnet, dass** eine erste Quenchzone zum Durchführen eines direkten Wärmetauschs zwischen einem Teilproduktstrom und einem ersten Quenchmedium umfasst wird, die stromabwärts der Wärmerückgewinnungsvorrichtung und stromaufwärts der Verbindungsvorrichtung angeordnet ist und dass ferner eine zweite Quenchzone zum Durchführen eines direkten Wärmetauschs zwischen dem Gesamtproduktstrom und einem zweiten Quenchmedium umfasst wird, die stromabwärts der Verbindungsvorrichtung und stromaufwärts des Verdichters angeordnet ist und die mit allen Teilprodukt-Leitungen der einzelnen Reaktorzüge in Fluidverbindung steht.

4. Anlage nach Anspruch 3, **dadurch gekennzeichnet, dass** die erste Quenchzone stromabwärts der Wärmerückgewinnungsvorrichtung und stromaufwärts der Absperrvorrichtung angeordnet ist.

5. Anlage nach einem der vorigen Ansprüche, **dadurch gekennzeichnet, dass** die mindestens eine Reaktionszone und die stromabwärts der Reaktionszone angeordnete Wärmerückgewinnungsvorrichtung in einem gemeinsamen Behälter angeordnet sind, wobei mittels der stromabwärts der Wärmerückgewinnungsvorrichtung angeordneten Teilprodukt-Leitung der Teilproduktstrom aus dem Behälter ausgeleitet wird.

6. Anlage nach einem der vorigen Ansprüche, **dadurch gekennzeichnet, dass**

   - die mindestens eine Reaktionszone und die stromabwärts der Reaktionszone angeordnete Wärmerückgewinnungsvorrichtung in einem gemeinsamen Behälter angeordnet sind,
   - mittels der stromabwärts der Wärmerückgewinnungsvorrichtung angeordneten Teilprodukt-Leitung der Teilproduktstrom aus dem Behälter ausgeleitet wird,
   - alle Teilprodukt-Leitungen der Reaktorzüge mittels der Verbindungsvorrichtung zusammengeführt werden,
   - die erste Quenchzone zwischen der Verbindungsvorrichtung und dem Verdichter angeordnet ist und mit allen Teilprodukt-Leitungen der einzelnen Reaktorzüge in Fluidverbindung steht.

7. Anlage nach einem der vorigen Ansprüche, **dadurch gekennzeichnet, dass**

   - die mindestens eine Reaktionszone, die stromabwärts der Reaktionszone angeordnete Wärmerückgewinnungsvorrichtung und die stromabwärts der Wärmerückgewinnungsvorrichtung angeordnete erste Quenchzone in einem gemeinsamen Behälter angeordnet sind,
   - mittels der stromabwärts der ersten Quenchzone angeordneten Teilprodukt-Leitung der Teilproduktstrom aus dem Behälter ausgeleitet wird,
   - alle Teilprodukt-Leitungen der Reaktorzüge mittels der Verbindungsvorrichtung zusammengeführt werden,
   - die zweite Quenchzone zwischen der Verbindungsvorrichtung und dem Verdichter angeordnet ist und mit allen Teilprodukt-Leitungen der einzelnen Reaktorzüge in Fluidverbindung steht.

8. Anlage nach einem der vorigen Ansprüche, **dadurch gekennzeichnet, dass**

   - die mindestens eine Reaktionszone, die stromabwärts der Reaktionszone angeordnete Wärmerückgewinnungsvorrichtung, die stromabwärts der Wärmerückgewinnungsvorrichtung angeordnete erste Quenchzone und die stromabwärts der ersten Quenchzone angeordnete zweite Quenchzone in einem gemeinsamen Behälter angeordnet sind,
   - mittels der stromabwärts der zweiten Quenchzone angeordneten Teilprodukt-Leitung der Teilproduktstrom aus dem Behälter ausgeleitet wird,
   - alle Teilprodukt-Leitungen der Reaktorzüge mittels der Verbindungsvorrichtung zusammengeführt werden.

9. Anlage nach Anspruch 5 bis 8, **dadurch gekennzeichnet, dass** der gemeinsame Behälter mit einer Leitung zum Ausleiten eines flüssigen Kondensats verbunden ist.

10. Anlage nach Anspruch 5 bis 9, **dadurch gekennzeichnet, dass** die Wärmerückgewinnungsvorrichtungen als Plattenwärmetauscher ausgestaltet sind.

11. Anlage nach Anspruch 10, **dadurch gekennzeichnet, dass** die Wärmerückgewinnungsvorrichtungen mindestens zwei Plattenwärmetauscher umfassen, die mit unterschiedlichen Kühlfluiden betrieben werden.

12. Anlage nach Anspruch 5 bis 11, **dadurch gekennzeichnet, dass** die Reaktionszonen in einer Wärmetransportbeziehung mit Plattenwärmetauschern stehen, mittels derer die Reaktionszonen durch indirekten Wärmetausch mit einem Kühlfluid gekühlt werden.

13. Verfahren zum Herstellen von Olefinen aus einem Oxygenate enthaltenden, fluiden Einsatzgemisch, umfassend folgende Schritte:

(a) Bereitstellen des Oxygenate enthaltenden, fluiden Einsatzgemischs,
(b) Zuführen des Oxygenate enthaltenden, fluiden Einsatzgemischs zu einer Anlage gemäß Anspruch 1 bis 12 und Umsetzen des Einsatzgemischs zu olefinhaltigen Teilproduktströmen unter Oxygenatumwandlungsbedingungen,
(c) Ausleiten mindestens einer olefinhaltiger Kohlenwasserstofffraktion aus der Anlage, wobei

- die Anlage mindestens zwei parallel angeordnete und parallel betreibbare Reaktorzüge umfasst, von denen
- mindestens ein Reaktorzug mit Oxygenate enthaltendem, fluiden Einsatzgemisch beaufschlagt wird, wobei der erhaltene Teilproduktstrom aus dem Reaktorzug ausgeleitet und über die geöffnete Absperrvorrichtung und die Verbindungsvorrichtung der Aufarbeitungsvorrichtung zugeführt wird,
und parallel dazu
- mindestens ein weiterer Reaktorzug mit einem gasförmigen sauerstoffhaltigen Regenerierungsmittel beaufschlagt wird, wobei ein Kohlenoxide enthaltendes Regenerierungsabgas erhalten wird, das aus dem Reaktorzug ausgeleitet wird und wobei die Absperrvorrichtung dieses Reaktorzuges geschlossen ist.

14. Verfahren nach Anspruch 13, **dadurch gekennzeichnet, dass** die Anlage drei Reaktorzüge umfasst, von denen zwei mit Oxygenate enthaltendem, fluiden Einsatzgemisch beaufschlagt werden und einer parallel dazu mit einem gasförmigen sauerstoffhaltigen Regenerierungsmittel beaufschlagt wird.

15. Verfahren nach Anspruch 13 oder 14, **dadurch gekennzeichnet, dass** mindestens 40 %, bevorzugt mindestens 70 % des Produktgases vor der Absperrvorrichtung als Kondensat abgeschieden werden.


**Claims**

1. Multi-strand plant for producing olefins from an oxygenates-containing fluid input mixture comprising the following constituents and assemblies in fluid connection with one another:

(a) at least two reactor trains arranged in parallel and operable in parallel, wherein each reactor train comprises:

(a1) at least one oxygenate-to-olefin (OTO) reaction zone containing a catalyst active and selective for the conversion of the oxygenates into olefins under oxygenate conversion conditions, means for supplying the oxygenates-containing input mixture to the reaction zone, means for discharging an olefins-containing partial product stream from the reaction zone, means for supplying a gaseous oxygen-containing regenerant to the reaction zone, means for discharging a carbon oxides-containing regeneration offgas from the reaction zone,
(a2) a thermal recovery apparatus arranged downstream of the reaction zone comprising at least one heat exchanger for performing indirect heat exchange between the product gas discharged from the reaction zone and a cooling fluid,
(a3) a partial product conduit arranged downstream of the thermal recovery apparatus for discharging the partial product stream from the reactor train,
(a4) a shutoff device in the conduit path of the partial product conduit for separating a reactor train from downstream plant parts and parallel reactor trains,

(b) a connecting device arranged downstream of the individual reactor trains for combining the individual partial product conduits into a complete product conduit,
(c) a compressor arranged downstream of the connecting device for compressing the complete product supplied using the complete product conduit,
(d) a multi-stage workup apparatus arranged downstream of the compressor for separating the compressed complete product into a plurality of olefin-containing hydrocarbon fractions,
(e) wherein furthermore at least one first quench zone for performing direct heat exchange between one or more partial product streams or the complete product stream and a first quenching medium is comprised, wherein the first quench zone is arranged downstream of the thermal recovery apparatus and upstream of the compressor.

2. Plant according to Claim 1, **characterized in that** arranged in every reactor train downstream of the thermal recovery apparatus are at least two partial product conduits for discharging the partial product stream from the reactor train which are arranged in parallel and operable in parallel, wherein at least one shutoff device is present in the conduit path of each of the parallel partial product conduits.

3. Plant according to Claim 1 or 2, **characterized in that** a first quench zone for performing direct heat exchange between a partial product stream and a first quenching medium is comprised and is arranged downstream of the thermal recovery apparatus and upstream of the connecting device and **in that** a second quench zone for performing direct heat exchange between the complete product stream and a second quenching medium is further comprised and is arranged downstream of the connecting device and upstream of the compressor and is in fluid connection with all partial product conduits from the individual reactor trains.

4. Plant according to Claim 3, **characterized in that** the first quench zone is arranged downstream of the thermal recovery apparatus and upstream of the shutoff device.

5. Plant according to any of the preceding claims, **characterized in that** the at least one reaction zone and the thermal recovery apparatus arranged downstream of the reaction zone are arranged in a common vessel, wherein the partial product conduit arranged downstream of the thermal recovery apparatus is used to discharge the partial product stream from the vessel.

6. Plant according to any of the preceding claims, **characterized in that**

   - the at least one reaction zone and the thermal recovery apparatus arranged downstream of the reaction zone are arranged in a common vessel,
   - the partial product stream is discharged from the vessel using the partial product conduit arranged downstream of the thermal recovery apparatus,
   - all partial product conduits of the reactor trains are combined using the connecting device,
   - the first quench zone is arranged between the connecting device and the compressor and is in fluid connection with all partial product conduits of the individual reactor trains.

7. Plant according to any of the preceding claims, **characterized in that**

   - the at least one reaction zone, the thermal recovery apparatus arranged downstream of the reaction zone and the first quench zone arranged downstream of the thermal recovery apparatus are arranged in a common vessel,
   - the partial product stream is discharged from the vessel using the partial product conduit arranged downstream of the first quench zone,
   - all partial product conduits of the reactor trains are combined using the connecting device,
   - the second quench zone is arranged between the connecting device and the compressor and is in fluid connection with all partial product conduits of the individual reactor trains.

8. Plant according to any of the preceding claims, **characterized in that**

   - the at least one reaction zone, the thermal recovery apparatus arranged downstream of the reaction zone, the first quench zone arranged downstream of the thermal recovery apparatus and the second quench zone arranged downstream of the first quench zone are arranged in a common vessel,
   - the partial product stream is discharged from the vessel using the partial product conduit arranged downstream of the second quench zone,
   - all partial product conduits of the reactor trains are combined using the connecting device.

9. Plant according to Claim 5 to 8, **characterized in that** the common vessel is connected to a conduit for discharging a liquid condensate.

**10.** Plant according to Claim 5 to 9, **characterized in that** the thermal recovery apparatuses are in the form of plate heat exchangers.

**11.** Plant according to Claim 10, **characterized in that** the thermal recovery apparatuses comprise at least two plate heat exchangers operated with different cooling fluids.

**12.** Plant according to Claim 5 to 11, **characterized in that** the reaction zones are in a heat transfer relationship with plate heat exchangers by means of which the reaction zones are cooled with a cooling fluid by indirect heat exchange.

**13.** Process for producing olefins from an oxygenates-containing fluid input mixture comprising the following steps:

(a) providing the oxygenates-containing fluid input mixture,
(b) supplying the oxygenates-containing fluid input mixture to a plant according to Claim 1 to 12 and converting the input mixture into olefin-containing partial product streams under oxygenate conversion conditions,
(c) discharging at least one olefin-containing hydrocarbon fraction from the plant, wherein

- the plant comprises at least two reactor trains arranged in parallel and operable in parallel, of which
- at least one reactor train is supplied with oxygenates-containing fluid input mixture, wherein the obtained partial product stream is discharged from the reactor train and via the opened shutoff device and the connecting device is supplied to the workup apparatus and in parallel therewith
- at least one further reactor train is supplied with a gaseous oxygen-containing regenerant, wherein a carbon oxides-containing regeneration offgas is obtained which is discharged from the reactor train and wherein the shutoff device of this reactor train is closed.

**14.** Process according to Claim 13, **characterized in that** the plant comprises three reactor trains, of which two are supplied with oxygenates-containing fluid input mixture and in parallel thereto one is supplied with a gaseous oxygen-containing regenerant.

**15.** Process according to Claim 13 or 14, **characterized in that** at least 40%, preferably at least 70%, of the product gas is separated as condensate upstream of the shutoff apparatus.

**Revendications**

**1.** Installation à plusieurs lignes permettant de produire des oléfines à partir d'un mélange d'alimentation fluide contenant des produits oxygénés, comprenant les composants et ensembles suivants en connexion fluidique les uns avec les autres :

(a) au moins deux trains de réacteurs agencés parallèlement et pouvant fonctionner en parallèle, dans laquelle chaque train de réacteurs comprend :

(a1) au moins une zone de réaction de produit oxygéné en oléfine (OTO), contenant un catalyseur actif et sélectif pour la conversion des produits oxygénés en oléfines sous des conditions de conversion de produits oxygénés, des moyens permettant d'acheminer le mélange d'alimentation contenant des produits oxygénés jusqu'à la zone de réaction, des moyens permettant d'évacuer un flux de produit partiel contenant des oléfines de la zone de réaction, des moyens permettant d'acheminer un régénérant gazeux contenant de l'oxygène jusqu'à la zone de réaction, des moyens permettant d'évacuer un effluent gazeux régénérant contenant du dioxyde de carbone de la zone de réaction,
(a2) un dispositif de récupération de chaleur agencé en aval de la zone de réaction, comprenant au moins un échangeur de chaleur permettant d'effectuer un échange de chaleur indirect entre le gaz produit évacué de la zone de réaction et un fluide de refroidissement,
(a3) une conduite de produit partiel agencée en aval du dispositif de récupération de chaleur et permettant d'évacuer le flux de produit partiel du train de réacteurs,
(a4) un dispositif d'arrêt dans la voie de conduite de la conduite de produit partiel permettant de séparer un train de réacteurs de parties d'installation placées en aval et de trains de réacteurs agencés parallèlement,

(b) un dispositif de connexion agencé en aval des trains de réacteurs individuels pour combiner les conduites de produit partiel individuelles en une conduite de produit total,

(c) un compresseur agencé en aval du dispositif de connexion et permettant de comprimer le produit total fourni au moyen de la conduite de produit total,

(d) un dispositif de retraitement à plusieurs étages agencé en aval du compresseur et permettant de séparer le produit total comprimé en plusieurs fractions hydrocarbonées contenant des oléfines,

(e) dans laquelle en outre au moins une première zone de refroidissement brusque permettant d'effectuer un échange de chaleur direct entre un ou plusieurs flux de produit partiel ou le flux de produit total et un premier agent de refroidissement brusque est prévue, la première zone de refroidissement brusque étant agencée en aval du dispositif de récupération de chaleur et en amont du compresseur.

2. Installation selon la revendication 1, **caractérisée en ce que**, dans chaque train de réacteurs, en aval du dispositif de récupération de chaleur, sont agencées au moins deux conduites de produit partiel agencées parallèlement et pouvant fonctionner en parallèle permettant d'évacuer le flux de produit partiel du train de réacteurs, au moins un dispositif d'arrêt étant présent dans la voie de conduite de chacune des conduites de produit partiel parallèles.

3. Installation selon la revendication 1 ou 2, **caractérisée en ce qu'**une première zone de refroidissement brusque permettant d'effectuer un échange de chaleur direct entre un flux de produit partiel et un premier agent de refroidissement brusque est prévue, laquelle est agencée en aval du dispositif de récupération de chaleur et en amont du dispositif de connexion, et **en ce qu'**en outre une deuxième zone de refroidissement brusque permettant d'effectuer un échange de chaleur direct entre le flux de produit total et un deuxième agent de refroidissement brusque est prévue, laquelle est agencée en aval du dispositif de connexion et en amont du compresseur et laquelle est en connexion fluidique avec toutes les conduites de produit partiel des trains de réacteurs individuels.

4. Installation selon la revendication 3, **caractérisée en ce que** la première zone de refroidissement brusque est agencée en aval du dispositif de récupération de chaleur et en amont du dispositif d'arrêt.

5. Installation selon l'une des revendications précédentes, **caractérisée en ce que** l'au moins une zone de réaction et le dispositif de récupération de chaleur agencé en aval de la zone de réaction sont agencés dans un récipient commun, le flux de produit partiel étant évacué du récipient au moyen de la conduite de produit partiel agencée en aval du dispositif de récupération de chaleur.

6. Installation selon l'une des revendications précédentes, **caractérisée en ce que**

- l'au moins une zone de réaction et le dispositif de récupération de chaleur agencé en aval de la zone de réaction sont agencés dans un récipient commun,
- le flux de produit partiel est évacué du récipient au moyen de la conduite de produit partiel agencée en aval du dispositif de récupération de chaleur,
- toutes les conduites de produit partiel des trains de réacteurs sont combinées au moyen du dispositif de connexion,
- la première zone de refroidissement brusque est agencée entre le dispositif de connexion et le compresseur et est en connexion fluidique avec toutes les conduites de produit partiel des trains de réacteurs individuels.

7. Installation selon l'une des revendications précédentes, **caractérisée en ce que**

- l'au moins une zone de réaction, le dispositif de récupération de chaleur agencé en aval de la zone de réaction et la première zone de refroidissement brusque agencée en aval du dispositif de récupération de chaleur sont agencés dans un récipient commun,
- le flux de produit partiel est évacué du récipient au moyen de la conduite de produit partiel agencée en aval de la première zone de refroidissement brusque,
- toutes les conduites de produit partiel des trains de réacteurs sont combinées au moyen du dispositif de connexion,
- la deuxième zone de refroidissement brusque est agencée entre le dispositif de connexion et le compresseur et est en connexion fluidique avec toutes les conduites de produit partiel des trains de réacteurs individuels.

8. Installation selon l'une des revendications précédentes, **caractérisée en ce que**

- l'au moins une zone de réaction, le dispositif de récupération de chaleur agencé en aval de la zone de réaction, la première zone de refroidissement brusque agencée en aval du dispositif de récupération de chaleur et la deuxième zone de refroidissement brusque agencée en aval de la première zone de refroidissement brusque sont agencés dans un récipient commun,

- le flux de produit partiel est évacué du récipient au moyen de la conduite de produit partiel agencée en aval de la deuxième zone de refroidissement brusque,
- toutes les conduites de produit partiel des trains de réacteurs sont combinées au moyen du dispositif de connexion.

9. Installation selon les revendications 5 à 8, **caractérisée en ce que** le récipient commun est connecté à une conduite permettant d'évacuer un condensat liquide.

10. Installation selon les revendications 5 à 9, **caractérisée en ce que** les dispositifs de récupération de chaleur sont configurés sous forme d'échangeurs de chaleur à plaques.

11. Installation selon la revendication 10, **caractérisée en ce que** les dispositifs de récupération de chaleur comprennent au moins deux échangeurs de chaleur à plaques qui fonctionnent avec des fluides de refroidissement différents.

12. Installation selon les revendications 5 à 11, **caractérisée en ce que** les zones de réaction sont dans une relation de transfert de chaleur avec des échangeurs de chaleur à plaques au moyen desquels les zones de réaction sont refroidies par échange de chaleur indirect avec un fluide de refroidissement.

13. Procédé de production d'oléfines à partir d'un mélange d'alimentation fluide contenant des produits oxygénés, comprenant les étapes suivantes :

(a) fournir le mélange d'alimentation fluide contenant des produits oxygénés,
(b) acheminer le mélange d'alimentation fluide contenant des produits oxygénés jusqu'à une installation selon les revendications 1 à 12 et convertir le mélange d'alimentation en flux de produit partiel contenant des oléfines sous des conditions de conversion de produits oxygénés,
(c) évacuer au moins une fraction hydrocarbonée contenant des oléfines de l'installation, dans lequel

- l'installation comprend au moins deux trains de réacteurs agencés parallèlement et pouvant fonctionner en parallèle, parmi lesquels
- au moins un train de réacteurs est exposé à un mélange d'alimentation fluide contenant des produits oxygénés, le flux de produit partiel obtenu étant évacué du train de réacteurs et étant acheminé au dispositif de retraitement via le dispositif d'arrêt ouvert et le dispositif de connexion, et en parallèle à cela
- au moins un autre train de réacteurs est exposé à un régénérant gazeux contenant de l'oxygène, un effluent gazeux régénérant contenant du dioxyde de carbone étant obtenu, lequel est évacué du train de réacteurs et le dispositif d'arrêt de ce train de réacteurs étant fermé.

14. Procédé selon la revendication 13, **caractérisé en ce que** l'installation comprend trois trains de réacteurs, parmi lesquels deux sont exposés à un mélange d'alimentation fluide contenant des produits oxygénés et un est exposé en parallèle à cela à un régénérant gazeux contenant de l'oxygène.

15. Procédé selon la revendication 13 ou 14, **caractérisé en ce qu'**au moins 40 %, de préférence au moins 70 % du gaz produit est séparé sous forme de condensat en amont du dispositif d'arrêt.

Fig. 1

Fig. 2

Fig. 3

Fig. 4

EP 3 733 633 B1

Fig. 5

EP 3 733 633 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2007140844 A1
- US 20150299067 A1
- US 4404414 A
- DE 102014112792 A1
- WO 2003051510 A2
- US 20110021857 A1